(19) Europäisches Patentamt
European Patent Office
Office européen des brevets

(11) Numéro de publication: 0 269 599 B1

(12) # FASCICULE DE BREVET EUROPEEN

(45) Date de publication de fascicule du brevet: **15.12.93**

(51) Int. Cl.[5]: **C07D 233/64**, A61K 31/415, A61K 31/415

(21) Numéro de dépôt: **87870149.9**

(22) Date de dépôt: **30.10.87**

Le dossier contient des informations techniques présentées postérieurement au dépôt de la demande et ne figurant pas dans le présent fascicule.

(54) **1H-Imidazoles substitués.**

(30) Priorité: **04.11.86 GB 8626287**

(43) Date de publication de la demande: **01.06.88 Bulletin 88/22**

(45) Mention de la délivrance du brevet: **15.12.93 Bulletin 93/50**

(84) Etats contractants désignés: **AT BE CH DE ES FR GB GR IT LI LU NL SE**

(56) Documents cités:
**EP-A- 0 024 829**
**EP-A- 0 058 047**

**CHEMICAL ABSTRACTS, vol. 106, no. 13, 30 mars 1987, page 20, résumé no. 95591n, Columbus, Ohio, US; J.M. SAVOLA: "Alfa-adrenoceptor activity of arylalkylimidazoles is improved by alfa-methylation and impaired by alfa-hydroxylation"**

**MODERN PHARMACOLOGY, C.R. Craig, R.E. Stitzel, 2nd Ed., Little, Brown and Co., 1986, page 368**

(73) Titulaire: **U C B, S.A.
326, Avenue Louise,
Bte 7
B-1050 Bruxelles(BE)**

(72) Inventeur: **Cossement, Eric
105 rue des Echevins
B-1050 Bruxelles(BE)**
Inventeur: **Geerts, Jean-Pierre
12, Habaru
B-6737 Leglise(BE)**
Inventeur: **Gobert, Jean
120, rue du Cornet
B-1040 Bruxelles(BE)**
Inventeur: **Michel, Philippe
54, rue Fr. Couteaux
B-1090 Bruxelles(BE)**

(74) Mandataire: **Dusseldorp, Raymond et al
UCB S.A.
Département D.T.B.
33, rue d'Anderlecht
B-1620 Drogenbos (BE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 269 599 B1

## Description

La présente invention se rapporte à de nouveaux 1H-imidazoles substitués, à leurs sels d'addition d'acides non toxiques pharmaceutiquement acceptables, à des procédés pour les préparer et à leur utilisation dans le domaine thérapeutique.

Elle concerne également les compositions thérapeutiques renfermant lesdits composés.

Selon le brevet européen n° 24.829, on connaît des 4-benzyl-1H-imidazoles dont le groupement benzyle porte sur le noyau phényle divers substituants choisis parmi l'hydrogène et les groupes chloro, bromo, fluoro, méthyle, éthyle, méthoxy, amino, hydroxyle et nitro. Ces composés possèdent les propriétés antihypertensive, antiulcéreuse, diurétique, sédative, analgésique, anti-inflammatoire et tranquillisante. Dans le brevet européen n° 58.047, on décrit des 4-(phénylalkyl)-1H-imidazoles similaires mais dans lesquels le radical alkyle du groupement phénylalkyle comporte 1 à 6 atomes de carbone; dans la plupart des composés, le noyau imidazole est en outre substitué par un radical alkyle contenant 1 à 7 atomes de carbone, un groupe phényle ou un radical benzyle substitué ou non. Ces composés possèdent des activités antithrombotique, antihypertensive, antimicrobienne et antifongique. Dans le brevet européen n° 72.615, on décrit aussi des 4-benzyl-1H-imidazoles similaires mais dans lesquels le groupement benzyle est substitué en position alpha par un radical alkyle. Le groupement benzyle porte sur le noyau phényle divers substituants choisis parmi l'hydrogène, un halogène, méthyle, éthyle, hydroxyle, méthoxy et le radical méthylènedioxy entre deux atomes de carbone adjacents. Les essais pharmacologiques décrits dans ce dernier brevet démontrent pour ces composés des propriétés antihypertensive, antithrombotique et diurétique. J.M. SAVOLA dans Naunyn-Schmiedeberg's Arch.Pharmacol.334,(1986),423-9 [Chem.Abstr.106,(1987),95591n], a étudié chez le rat les activités hypotensives et bradycardiques de quelques 4-(phénylalkyl)-1H-imidazoles dont le noyau phényle est substitué en positions 2-, 2,3- ou 2,6- par des radicaux méthyles. On montre entre autres dans cette étude que l'introduction d'un radical hydroxyle en position alpha sur le pont alkyle situé entre les noyaux imidazole et phényle, entraîne toujours un diminution des activités hypotensives et bradycardiques.

Il n'est toutefois pas fait mention dans les documents précités de propriétés anti-ischémiques.

La présente invention procure de nouveaux 1H-imidazoles substitués doués d'excellentes propriétés anti-ischémiques cardiaque, cérébrale et tissulaire. Ces composés peuvent donc être utilisés entre autres pour la prévention et le traitement des troubles induits par les ischémies en général. Parmi ces troubles, l'angor est l'expression clinique d'une ischémie myocardique aiguë qui est le résultat d'un déséquilibre momentané entre la demande en oxygène du myocarde et la fourniture en oxygène par la circulation coronaire, déséquilibre pouvant conduire dans les cas graves à l'infarctus du myocarde. C'est pourquoi, les composés conformes à l'invention conviennent particulièrement bien pour le traitement de l'angor et de l'infarctus du myocarde. En outre, on a trouvé que certains de ces composés possèdent une activité hypotensive non négligeable.

Les nouveaux composés de la présente invention sont des 1H-imidazoles substitués répondant à la formule générale

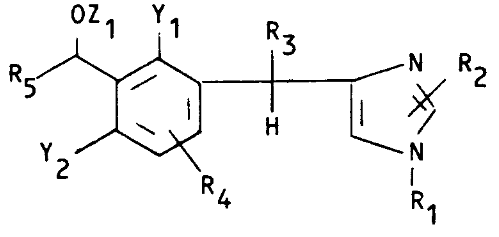

(I)

dans laquelle

$R_1$, $R_2$, $R_3$ et $R_5$, qui peuvent être identiques ou différents, représentent chacun un atome d'hydrogène ou un radical alkyle contenant 1 à 4 atomes de carbone,

$R_4$ représente un atome d'hydrogène, un radical alkyle contenant 1 à 4 atomes de carbone ou un radical alkoxy contenant 1 à 4 atomes de carbone,

un des radicaux $Y_1$ et $Y_2$ représente de l'hydrogène et l'autre un radical $OZ_2$, et

$Z_1$ et $Z_2$ pris isolément représentent tous deux de l'hydrogène ou un radical alkyle contenant 1 à 4 atomes de carbone et ensemble un groupe $-CH_2-$ ou $-C(CH_3)_2-$,

ainsi que leurs sels d'addition d'acides non toxiques pharmaceutiquement acceptables.

Les composés de formule I peuvent se présenter soit sous la forme racémique, soit sous forme de l'un ou l'autre énantiomère lorsque la molécule comporte un seul atome de carbone asymétrique. Lorsque la molécule comporte deux atomes de carbone asymétriques, ces composés se présentent généralement sous la forme d'un mélange des diastéréoisomères. Ces diverses formes entrent également dans le cadre de la présente invention.

Lorsque dans la formule I, les symboles $Z_1$ et $Z_2$ représentent chacun un atome d'hydrogène, un représentant spécifique des composés de l'invention est par exemple le 3-[(1H-imidazol-4-yl)méthyl]-2-hydroxybenzèneméthanol, tandis que lorsque dans la formule I, les symboles $Z_1$ et $Z_2$ représentent chacun un radical alkyle en $C_1$-$C_4$, un composé spécifique conforme à l'invention est par exemple le 4-[(2,6-diméthoxy-3-méthoxyméthylphényl)méthyl]-1H-imidazole.Dans le cas où les symboles $Z_1$ et $Z_2$ représentent ensemble un groupe $-CH_2-$ ou $-C(CH_3)_2-$, on peut citer comme exemples spécifiques de composés conformes à l'invention le 4-[(4H-1,3-benzodioxin-8-yl)méthyl]-1H-imidazole ($Y_1$ = $OZ_2$, $Y_2$ = H), le 4-[(4H-1,3-benzodioxin-6-yl)méthyl]-1H-imidazole ($Y_1$ = H, $Y_2$ = $OZ_2$) et le 4-[1-(2,2-diméthyl-4H-1,3-benzodioxin-8-yl)éthyl]-1H-imidazole ($Y_1$ = $OZ_2$, $Y_2$ = H).

Les composés préférés de la présente invention sont les 1H-imidazoles répondant à la formule générale I, dans laquelle

| | |
|---|---|
| $R_1$, $R_2$, $R_4$ et $R_5$ | représentent de l'hydrogène, |
| $R_3$ | représente de l'hydrogène ou un radical alkyle contenant 1 à 4 atomes de carbone, et |
| $Z_1$ et $Z_2$ | pris isolément représentent de l'hydrogène et ensemble un groupe $-CH_2-$ ou $-C(CH_3)_2-$, |

ainsi que leurs sels d'addition d'acides non toxiques pharmaceutiquement acceptables.

Les composés particulièrement préférés conformes à l'invention sont:

- le 3-[(1H-imidazol-4-yl)méthyl]-2-hydroxybenzèneméthanol;
- le 3-[(1H-imidazol-4-yl)méthyl]-6-hydroxybenzèneméthanol;
- le 3-[1-(1H-imidazol-4-yl)éthyl]-2-hydroxybenzèneméthanol;
- le 3-[1-(1H-imidazol-4-yl)pentyl]-2-hydroxybenzèneméthanol;
- le 4-[(4H-1,3-benzodioxin-8-yl)méthyl]-1H-imidazole et son chlorhydrate;
- le 4-[1-(4H-1,3-benzodioxin-8-yl)éthyl]-1H-imidazole;
- le 4-[(2,2-diméthyl-4H-1,3-benzodioxin-8-yl)méthyl]-1H-imidazole;
- le 4-[1-(2,2-diméthyl-4H-1,3-benzodioxin-8-yl)éthyl]-1H-imidazole;
- le 4-[1-(2,2-diméthyl-4H-1,3-benzodioxin-8-yl)pentyl]-1H-imidazole.

La présente invention concerne également les sels d'addition d'acides non toxiques pharmaceutiquement acceptables des 1H-imidazoles de formule I. Comme exemples d'acides pharmaceutiquement acceptables, on peut citer les acides minéraux comme les acides chlorhydrique, bromhydrique, sulfurique, nitrique, phosphorique, etc. et les acides organiques comme les acides acétique, citrique, tartrique, benzoïque, salicylique, maléique, etc.

Les 1H-imidazoles substitués de formule I, dans laquelle $Z_1$ et $Z_2$ pris isolément représentent un radical alkyle en $C_1$-$C_4$ et ensemble un groupe $-CH_2-$ ou $-C(CH_3)_2-$, sont préparés par réduction, selon les méthodes conventionnelles, d'un composé imidazolique de formule

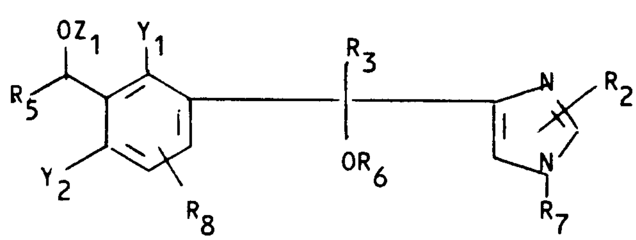 (II)

dans laquelle $R_2$, $R_3$ et $R_5$ ont la signification donnée plus haut, $R_6$ représente un atome d'hydrogène ou un radical alkyle ayant 1 à 4 atomes de carbone, $R_7$ représente un radical $R_1$ tel que défini plus haut ou un radical aisément séparable par réduction, tel qu'un radical benzyle ou triphénylméthyle, $R_8$ représente un radical $R_4$ tel que défini plus haut ou un atome de chlore, un des radicaux $Y_1$ et $Y_2$ représente de l'hydrogène et l'autre un radical $OZ_2$, $Z_1$ et $Z_2$ ayant la signification donnée plus haut.

Cette réduction est généralement effectuée soit dans l'acide acétique glacial ou dans le méthanol, en présence d'un catalyseur, tel que par exemple du charbon palladié, à une température de 20 à 80 °C, de préférence environ 80 °C, et sous atmosphère d'hydrogène à une pression comprise entre 2 et 5 bars, soit

dans l'ammoniac liquide, en présence d'un métal alcalin comme le lithium ou de préférence le sodium, et d'un solvant auxiliaire tel que le toluène ou un éther comme par exemple le tétrahydrofuranne. Dans le cas où un atome de chlore est présent sur le noyau phényle des composés de départ de formule II ($R_8$ = Cl), cet atome de chlore est éliminé au cours de la réduction.

Les 1H-imidazoles substitués de formule I, dans laquelle $Z_1$ et $Z_2$ sont de l'hydrogène, peuvent être préparés par hydrolyse en milieu acide aqueux d'un 4-[[2,2-diméthyl-4H-1,3-benzodioxin-6(ou 8)-yl]méthyl]-1H-imidazole de formule

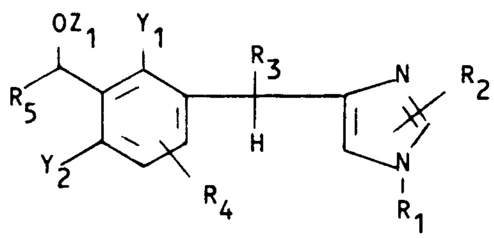

(III)

dans laquelle $R_1$, $R_2$, $R_3$, $R_4$ et $R_5$ ont la signification donnée plus haut, un des radicaux $Y_1$ et $Y_2$ représente de l'hydrogène et l'autre un radical $OZ_2$, $Z_1$ et $Z_2$ étant ensemble le groupe $-C(CH_3)_2-$.

Cette hydrolyse est effectuée au moyen d'un acide minéral tel que l'acide chlorhydrique aqueux (pH 2 à 3), à une température comprise entre 20 et 100 °C pendant une à deux heures environ.

Le milieu réactionnel est ensuite alcalinisé jusqu'à pH 8 à 8,6 au moyen d'une base minérale telle que l'hydroxyde de sodium et le produit de la réaction est récupéré par filtration. Il est lavé à l'eau pour le débarrasser des sels minéraux et recristallisé dans un solvant approprié.

Selon une variante, les 1H-imidazoles substitués de formule I, dans laquelle $Y_1$ = $OZ_2$ et $Y_2$, $Z_1$, $Z_2$ et $R_5$ sont de l'hydrogène, peuvent également être préparés par réduction, selon les méthodes conventionnelles, d'un 3-[(1H-imidazol-4-yl)méthyl]-2-hydroxybenzoate d'alkyle de formule

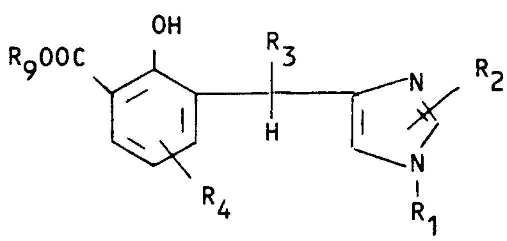

(IV)

dans laquelle $R_1$, $R_2$, $R_3$ et $R_4$ ont la signification donnée ci-dessus et $R_9$ représente un radical alkyle contenant 1 à 4 atomes de carbone, de préférence méthyle ou éthyle.

Cette réduction peut être effectuée au moyen de divers hydrures, de préférence l'hydrure de lithium-aluminium, à la température de reflux dans un solvant organique inerte comme par exemple le tétrahydrofuranne. Ce dernier a l'avantage de donner une réaction rapide, propre et complète.

Les sels d'addition d'acides non toxiques pharmaceutiquement acceptables peuvent être préparés à partir des 1H-imidazoles de formule I par des méthodes connues en soi.

Les composés de départ de formule II, dans laquelle $R_6$ = H et $R_7$ représente un radical alkyle en $C_1$-$C_4$ ou un radical aisément séparable par réduction, et qui sont aussi des composés nouveaux, peuvent être préparés selon l'un ou l'autre des deux procédés suivants:

a) on fait réagir un dérivé organométallique de formule V avec un 4-($R_3$-CO)-1H-imidazole de formule VI, selon l'équation

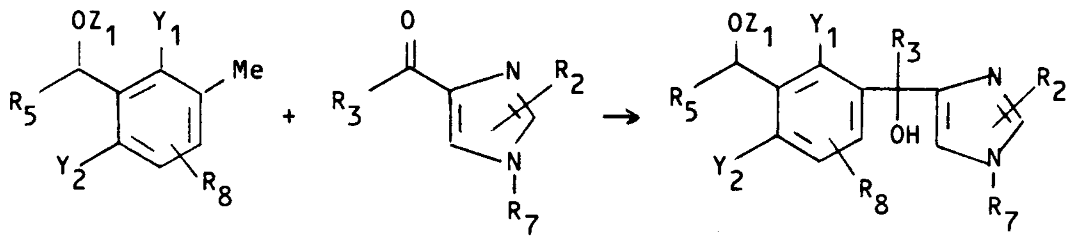

(V) (VI) (II) avec $R_6$ = H et $R_7$ différent de H

dans ces formules $R_2$, $R_3$, $R_5$, $R_7$, $R_8$, $Y_1$, $Y_2$, $Z_1$ et $Z_2$ ayant la signification donnée plus haut, $R_7$ étant autre que de l'hydrogène et Me représentant MgBr ou Li.

b) on fait réagir une cétone de formule VII avec un composé organomagnésien de formule VIII selon l'équation

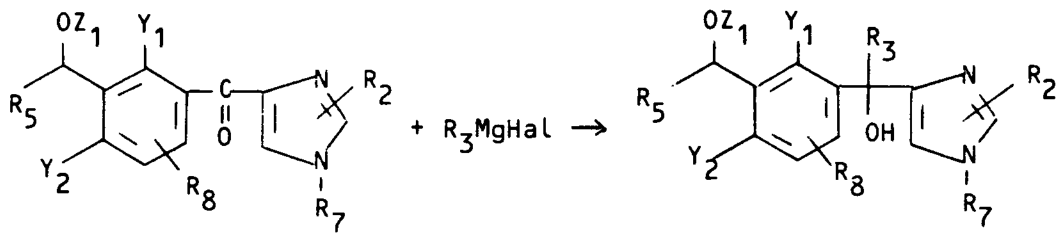

(VII) (VIII) (II) avec $R_6$ = H et $R_7$ différent de H

dans ces formules $R_2$, $R_5$, $R_7$, $R_8$, $Y_1$, $Y_2$, $Z_1$ et $Z_2$ ayant la signification donnée plus haut, $R_3$ étant un radical alkyle en $C_1$-$C_4$, $R_7$ étant autre que l'hydrogène et Hal représentant un atome d'halogène.

Les procédés a) et b) ci-dessus sont de préférence réalisés dans le tétrahydrofuranne sous atmosphère inerte. Dans le cas des composés dans lesquels $Z_1$ et $Z_2$ représentent ensemble le groupe $-CH_2-$, la température à laquelle cette réaction exothermique s'effectue peut atteindre 50 °C. Dans les cas des composés dans lesquels $Z_1$ et $Z_2$ pris isolément représentent un radical alkyle en $C_1$-$C_4$ et ensemble le groupe $-C(CH_3)_2-$, plus sensibles à la chaleur, la température de la réaction sera généralement maintenue entre 0 et 20 °C et en tous cas ne dépassera pas 40 °C.

Les composés de formule II préparés selon les procédés a) et b) ci-dessus et dans lesquels $R_8$ représente un atome de chlore, peuvent éventuellement être transformés en les composés correspondants de formule II dans laquelle $R_8$ représente de l'hydrogène, par réduction, selon les méthodes conventionnelles.

Les dérivés organométalliques de formule V sont obtenus de manière connue en soi par réaction du magnésium ou du lithium sur les dérivés bromés correspondants. Dans le cas des composés dans lesquels $Z_1$ et $Z_2$ représentent ensemble le groupe $-CH_2-$, la température de formation du dérivé organométallique de formule V peut atteindre 50 °C. Dans le cas des composés dans lesquels $Z_1$ et $Z_2$ pris isolément représentent un radical alkyle en $C_1$-$C_4$ et ensemble le groupe $-C(CH_3)_2-$, cette température sera maintenue en dessous de 40 °C.

Lorsque les dérivés bromés sont des 4H-1,3-benzodioxines bromées ($Z_1$ et $Z_2$ = $-CH_2-$), ils peuvent être préparés en faisant réagir le paraformaldéhyde avec un bromophénol approprié, tandis que lorsque ces dérivés sont des 2,2-diméthyl-4H-1,3-benzodioxines bromées ($Z_1$ et $Z_2$ = $-C(CH_3)_2-$), ils peuvent être obtenus par réaction du 2,2-diméthoxypropane avec un 2-hydroxybenzèneméthanol bromé, convenablement substitué, lui-même préparé à partir d'un bromophénol approprié et d'une solution aqueuse de formaldéhyde.

Quant aux dérivés bromés dans lesquels $Z_1$ et $Z_2$ représentent un radical alkyle en $C_1$-$C_4$, ils peuvent être préparés par alkylation, selon des méthodes connues en soi, des bromophénols correspondants.

Les 4-($R_3$-CO)-1H-imidazoles de formule VI peuvent être obtenus, d'une manière générale, par oxydation des 1H-imidazole-4-méthanols correspondants au moyen de $MnO_2$ activé selon la méthode décrite par J.L. KELLEY, C.A. MILLER et E.W. McLEAN (J.Med.Chem.20,(1977),721-723).

Les cétones de formule VII sont préparées par oxydation d'un alcool correspondant de formule II, dans laquelle $R_3$ et $R_6$ sont de l'hydrogène, par exemple par action de l'oxyde de manganèse dans un solvant chloré, de préférence le chloroforme, à l'ébullition, l'alcool de départ de formule II étant lui-même préparé par le procédé a) décrit plus haut.

Quant aux composés organomagnésiens de formule VIII, ils sont obtenus de manière connue en soi en faisant réagir du magnésium sur l'halogénure d'alkyle correspondant.

Les composés de départ de formule II dans laquelle $R_6$ et $R_7$ sont de l'hydrogène, qui sont également des composés nouveaux, sont préparés par réduction d'un composé correspondant de formule II dans laquelle $R_6$ représente un atome d'hydrogène et $R_7$ un radical aisément séparable par réduction (p.ex. benzyle ou triphénylméthyle). Cette réduction peut être réalisée dans l'ammoniac liquide en présence de sodium. Toutefois, il est malaisé de suivre l'évolution de la réaction et dès lors, il est préférable d'effectuer une hydrogénolyse en présence de charbon palladié, dans un alcool, à une température pouvant varier entre 20 °C et 80 °C et sous atmosphère d'hydrogène à une pression comprise entre 2 et 5 bars.

Quant aux composés de départ de formule II dans laquelle $R_6$ représente un radical alkyle en $C_1$-$C_4$ et $R_7$ de l'hydrogène, on les obtient par réduction dans un alcool de formule $R_6OH$ ($R_6$ = alkyle en $C_1$-$C_4$) d'un composé correspondant de formule II dans laquelle $R_6$ représente un atome d'hydrogène et $R_7$ un radical aisément séparable par réduction.

Dans la plupart des cas, cette réduction ne conduit pas uniquement au composé alkoxylé de formule II ($R_6$ = alkyle, $R_7$ = H) souhaité, mais on obtient un mélange de ce composé alkoxylé et de l'alcool correspondant de formule II ($R_6$ = $R_7$ = H). Ce mélange est généralement utilisé tel quel pour effectuer l'étape suivante d'élimination du groupement $OR_6$ par réduction. Toutefois, si on le désire, l'alcool de formule II ($R_6$ = $R_7$ = H) et le composé alkoxylé de formule II ($R_6$ = alkyle, $R_7$ = H) peuvent être isolés à l'état pur, par exemple, par chromatographie du mélange.

En outre, les chlorhydrates des alcools de formule II ($R_6$ = $R_7$ = H) peuvent être transformés aisément en les chlorhydrates des composés alkoxylés de formule II ($R_6$ = alkyle, $R_7$ = H) par simple chauffage dans l'alcool correspondant de formule $R_6OH$.

Lorsqu'un atome de chlore est présent sur le noyau phényle des composés de départ de formule II ($R_8$ = Cl), il est généralement éliminé en même temps que le radical $R_7$ aisément séparable au cours des procédés de réduction qui viennent d'être décrits.

Les 4-[[2,2-diméthyl-4H-1,3-benzodioxin-6(ou 8)-yl]méthyl]-1H-imidazoles de formule III sont préparés par réduction, selon les méthodes conventionnelles, d'un composé de formule II, dans laquelle $R_2$, $R_3$, $R_5$, $R_6$, $R_7$, $R_8$, $Y_1$ et $Y_2$ ont la signification donnée plus haut et $Z_1$ et $Z_2$ représentent ensemble le groupe -C-$(CH_3)_2$-.

Cette réduction est généralement effectuée soit dans l'acide acétique glacial ou dans le méthanol, en présence d'un catalyseur, tel que par exemple du charbon palladié, à une température de 20 à 80 °C, de préférence environ 80 °C, et sous atmosphère d'hydrogène à une pression comprise entre 2 et 5 bars, soit dans l'ammoniac liquide, en présence d'un métal alcalin comme le lithium ou de préférence le sodium, et d'un solvant auxiliaire tel que le toluène ou un éther comme par exemple le tétrahydrofuranne. L'atome de chlore éventuellement présent sur le noyau phényle des composés de départ de formule II ($R_8$ = Cl) est éliminé au cours de cette réduction.

Les 3-[(1H-imidazol-4-yl)méthyl]-2-hydroxybenzoates d'alkyle de départ de formule IV peuvent être préparés par un procédé en plusieurs stades:

(a) réaction en présence d'éthylate de sodium d'un 2-oxo-cyclohexanecarboxylate d'alkyle de formule IX avec un 4-chlorométhyl-1H-imidazole de formule X, pour obtenir un 1-[(1H-imidazol-4-yl)méthyl]-2-oxo-cyclohexanecarboxylate d'alkyle de formule XI, selon l'équation

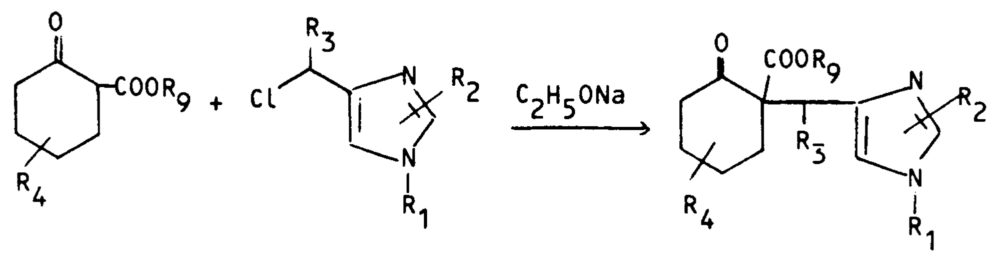


(IX) (X) (XI)

(b) chauffage pendant plusieurs heures à une température voisine de 100 à 105 °C, en présence d'un excès d'éthylate de sodium du 1-[(1H-imidazol-4-yl)méthyl]-2-oxo-cyclohexanecarboxylate d'alkyle de formule XI, ce qui conduit par réarrangement de la molécule, au 3-[(1H-imidazol-4-yl)méthyl]-2-oxo-cyclohexanecarboxylate d'alkyle de formule XII, selon l'équation

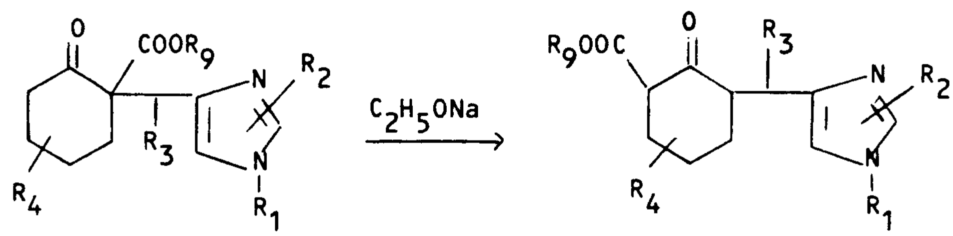


(XI) (XII)

(c) oxydation par le brome dans l'acide acétique à la température du reflux pendant plusieurs heures du 3-[(1H-imidazol-4-yl)méthyl]-2-oxo-cyclohexanecarboxylate d'alkyle de formule XII, pour obtenir un mélange de 3-[(1H-imidazol-4-yl)méthyl]-2-hydroxybenzoate d'alkyle et de ses dérivés bromés sur les positions 2 et/ou 5 de l'imidazole, puis hydrogénolyse en présence de palladium sur carbone et de rhodium sur carbone, sous une pression d'hydrogène de 3,5 bars, du mélange obtenu, ce qui conduit finalement au 3-[(1H-imidazol-4-yl)méthyl]-2-hydroxybenzoate d'alkyle de formule IV, selon l'équation

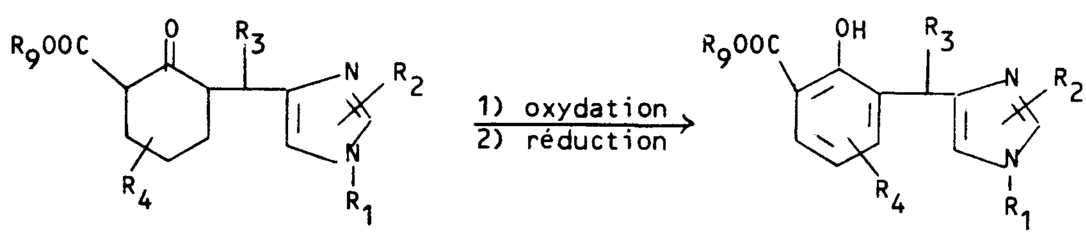


(XII) (IV)

dans ces formules $R_1$, $R_2$, $R_3$, $R_4$ ayant la signification donnée plus haut et $R_9$ représentant un radical alkyle en $C_1$-$C_4$, de préférence méthyle ou éthyle.

Les 4-chlorométhyl-1H-imidazoles de formule X peuvent être préparés à partir des 1H-imidazole-4-méthanols correspondants par chloruration selon des méthodes connues en soi.

Comme il a été indiqué plus haut, les 1H-imidazoles substitués de formule I ainsi que leurs sels d'addition d'acides non toxiques pharmaceutiquement acceptables possèdent d'excellentes propriétés anti-ischémiques cardiaque, cérébrale et tissulaire. Les essais pharmacologiques décrits ci-après mettent en évidence ces propriétés précieuses.

Les produits suivants de la présente invention ont été soumis à des essais pharmacologiques:

- 3-[(1H-imidazol-4-yl)méthyl]-2-hydroxybenzèneméthanol (produit A),
- 3-[(1H-imidazol-4-yl)méthyl]-2-hydroxy-5-méthyl-benzèneméthanol (produit B),
- 3-[(1H-imidazol-4-yl)méthyl]-6-hydroxybenzèneméthanol (produit C),

- chlorhydrate de 4-[(6-méthyl-4H-1,3-benzodioxin-8-yl)méthyl]-1H-imidazole (produit D),
- chlorhydrate de 4-[(4H-1,3-benzodioxin-8-yl)méthyl]-1H-imidazole (produit E),
- 4-[(2,2-diméthyl-4H-1,3-benzodioxin-8-yl)méthyl]-1H-imidazole(produit F),
- 4-[(2,2,6-triméthyl-4H-1,3-benzodioxin-8-yl)méthyl]-1H-imidazole (produit G),
- 4-[1-(4H-1,3-benzodioxin-8-yl)éthyl]-1H-imidazole (produit H),
- 4-[1-(2,2-diméthyl-4H-1,3-benzodioxin-8-yl)pentyl]-1H-imidazole (produit I),
- 3-[1-(1H-imidazol-4-yl)pentyl]-2-hydroxybenzèneméthanol (produit J),
- chlorhydrate de 4-[[2,6-diméthoxy-3-(1-méthoxyéthyl)phényl]méthyl]-1H-imidazole (produit K),
- 4-[1-(2,2-diméthyl-4H-1,3-benzodioxin-8-yl)éthyl]-1H-imidazole (produit L),
- 3-[1-(1H-imidazol-4-yl)éthyl]-2-hydroxybenzèneméthanol (produit M),
- d-4-[1-(4H-1,3-benzodioxin-8-yl)éthyl]-1H-imidazole (produit N).

1. Activité anti-ischémique cardiaque.

1.1 Chez le rat.

Chez le rat anesthésié et thoracotomisé, une ligature coronaire induit une ischémie cardiaque stable (voir H. SELYE et al., Angiology,11,(1960),398-407) qui se traduit par une élévation de l'onde R de l'électrocardiogramme épicardique (voir L.G.T. RIBEIRO et al., J.Electrocardiol.12,(1979),89-95). L'action anti-ischémique d'un produit se manifeste par une diminution de l'élévation de l'onde R.

Le tableau I montre pour les produits soumis à l'essai la dose ($DE_{20}$ en mg/kg) qui, administrée par voie intraveineuse à un groupe de 5 rats, produit une diminution moyenne d'au moins 20% de l'élévation de l'onde R sur l'ensemble des animaux. Comme substances de référence, on utilise les produits suivants:

- propranolol : 1-(isopropylamino)-3-(1-naphtyloxy)-2-propanol;
- vérapamil : alpha-[3-[[2-(3,4-diméthoxyphényl)éthyl] méthylamino]propyl]-3,4-diméthoxy-alpha-(1-méthyléthyl)benzèneacétonitrile;
- nifédipine : ester diméthylique de l'acide 1,4-dihydro-2,6-diméthyl-4-(2-nitrophényl)-3,5-pyridinedicarboxylique.

TABLEAU I

| Produits | $DE_{20}$ (en mg/kg) |
|---|---|
| A | 0,006 |
| B | 0,22 |
| C | 0,20 |
| D | 0,09 |
| E | 0,25 |
| F | 0,24 |
| G | 0,26 |
| J | 0,26 |
| L | 0,03 |
| M | 0,02 |
| N | < 0,23 |
| propranolol | 9,47 |
| vérapamil | 0,32 |
| nifédipine | 1,70 |

1.2 Chez le chien.

Chez le chien éveillé porteur d'un obturateur (occluder) pneumatique coronaire, l'occlusion coronaire provoque une élévation du segment ST de l'électrocardiogramme épicardique. L'action anti-ischémique d'un composé se manifeste par une réduction de l'élévation du segment ST (P.R. MAROKO et E. BRAUNWALD, Circ.53, (1976,Suppl.I),162-168; S.E. EPSTEIN et al., Circ.53,-(1976,Suppl.I), 191-197). Le tableau II montre pour les produits soumis à l'essai la dose ($DE_{20}$ en mg/kg) qui, administrée par voie intraveineuse à un groupe de 10 animaux, produit une diminution moyenne d'au moins 20% de l'élévation du segment ST sur l'ensemble des animaux.

TABLEAU II

| Produits | $DE_{20}$ (en mg/kg) |
|---|---|
| A | 0,002 |
| B | 0,02 |
| C | 0,02 |
| D | 0,09 |
| E | 0,01 |
| F | 0,24 |
| G | 0,26 |
| H | 0,007 |
| I | 0,1 |
| J | 0,03 |
| K | 0,31 |
| L | 0,008 |
| M | 0,0007 |
| propranolol | 0,53 |

De ces tableaux, il ressort que les produits de l'invention sont bien actifs et supérieurs aux substances de référence.

2. Activité anti-ischémique cérébrale.

La ligature des deux carotides chez des souris anesthésiées entraîne progressivement la mort d'au moins la moitié des animaux après 90 minutes.

Dans cet essai, on administre par voie intrapéritonéale à un groupe de 18 animaux, 30 minutes avant d'effectuer les ligatures, une dose de 0,2 mg/kg du composé A dissous dans 10 ml de sérum physiologique. Un groupe témoin composé également de 18 animaux, ne reçoit au même moment que du sérum physiologique (solution aqueuse de chlorure de sodium à 0,9%). On note ensuite, 30 minutes et 90 minutes après les ligatures, le nombre d'animaux survivants dans le groupe témoin et dans le groupe traité. Les résultats obtenus sont consignés dans le tableau III.

**TABLEAU III**

| Produit | Nombre d'animaux survivants | | Durée (min.) |
|---|---|---|---|
| | groupe témoin | groupe traité | |
| A | 12/18 | 17/18 | 30 |
| A | 9/18 | 15/18 | 90 |

Ce tableau montre que le composé A augmente significativement le nombre d'animaux survivants dans le groupe traité par le composé A par rapport au nombre d'animaux survivants dans le groupe témoin. La protection réalisée et calculée selon la formule d'ABBOTT (voir FINNEY, Statist.Meth.in Biol.Assay, 2nd Ed. 1964) est de 66% après 90 minutes.

3. Activité anti-ischémique tissulaire.

L'accumulation d'ions $Ca^{++}$ dans les tissus ischémiés est, en physiopathologie animale, une indication de l'état de souffrance des cellules (voir A.D. SHARMA et al., J.Clin.Invest.72,(1983),802-818). Une occlusion coronaire de longue durée (150 minutes) pratiquée sur des rats anesthésiés, provoque une accumulation d'ions calciques (+ 250%) dans les tissus de la zone nécrosée.

Dans cet essai, on administre le composé étudié, en l'espace d'une minute, dix minutes avant l'occlusion coronaire, à un groupe d'au moins 6 rats, par voie intraveineuse à la dose de 100 myg/kg dissous dans 0,8 ml de sérum physiologique. Cette administration est suivie immédiatement d'une perfusion lente du composé testé à la dose de 100 myg/kg par heure pendant 2 heures et demi. Après avoir disséqué la zone nécrosée, on y mesure l'accumulation tissulaire de $Ca^{++}$ par spectrométrie d'absorption atomique selon le procédé décrit par Hideto OHHARA et al. (J.Mol.Cell.Cardiol.14,(1982),13-20). Un groupe témoin composé d'au moins 6 rats, ne reçoit que du sérum physiologique.

L'activité du produit A est comparée à celle du vérapamil administré dans les mêmes conditions; on utilise toutefois une dose de 320 myg/kg de vérapamil au lieu de 100 myg/kg.

Dans le tableau IV ci-dessous, on indique la diminution en % de la moyenne des concentrations d'ions $Ca^{++}$ dans les tissus ischémiés du groupe traité par rapport à la moyenne des concentrations d'ions $Ca^{++}$ dans les tissus ischémiés du groupe témoin. Les résultats montrent que, bien qu'étant administré à une dose 3 fois plus faible que celle du vérapamil, le composé A est plus de 2 fois plus actif pour combattre l'infiltration du calcium dans les tissus de la zone nécrosée.

**TABLEAU IV**

| Produits | Diminution de la concentration de $Ca^{++}$ tissulaire (en %) |
|---|---|
| vérapamil | 18 |
| A | 40 |

4. Toxicité.

La toxicité des produits de l'invention a été déterminée chez la souris mâle NMRI au moyen du test d'Irwin (S. IRWIN, Psychopharmacologia,13,(1968),222-257).

Des doses progressives du produit étudié sont administrées par voie intrapéritonéale à des groupes de trois souris jusqu'à ce que la dose mortelle soit atteinte (dose provoquant la mort de deux animaux sur trois dans les 48 heures).

Dans le tableau V ci-dessous, on donne la dose mortelle observée pour les produits de l'invention. Il ressort de ce tableau que les produits de l'invention sont très peu toxiques, la dose mortelle se situant bien au-dessus des doses actives.

**TABLEAU V**

| Produits | Dose mortelle (en mg/kg) |
|---|---|
| A | 204 |
| B | > 218 |
| C | 613 |
| D | 80 |
| E | 253 |
| F | 73 |
| G | 258 |
| H | 69 |
| I | 300 |

**TABLEAU V (suite)**

| Produits | Dose mortelle (en mg/kg) |
|---|---|
| J | 260 |
| K | 31,2 |
| L | 25,8 |

Les compositions pharmaceutiques renfermant les produits de l'invention peuvent être administrées par

voie orale, parentérale ou rectale.

Les compositions pharmaceutiques utilisables pour l'administration orale peuvent être solides ou liquides, par exemple sous forme de comprimés (enrobés ou non), pilules, dragées, capsules en gélatine, solutions, sirops, etc. De même, les compositions utilisables pour l'administration par voie parentérale, sont les formes pharmaceutiquement connues pour ce genre d'administration, par exemple des solutions, des suspensions ou émulsions aqueuses ou huileuses. Pour l'administration par voie rectale, les compositions contenant les produits de l'invention se présentent généralement sous forme de suppositoires.

Les formes pharmaceutiques telles que solutions injectables, suspensions injectables, comprimés, gouttes, suppositoires, etc. sont préparées selon les méthodes couramment utilisées par les pharmaciens. On mélange les composés de l'invention avec un véhicule solide ou liquide, non toxique, pharmaceutiquement acceptable, et éventuellement avec un agent dispersant, un agent désintégrant, un agent stabilisant, etc. On peut y ajouter, le cas échéant, des agents édulcorants, des agents colorants, etc.

Le pourcentage de produit actif dans les compositions pharmaceutiques peut varier dans des limites très larges, selon le patient et le mode d'administration, en particulier selon la fréquence d'administration. Quant à la posologie journalière, elle peut varier dans une gamme étendue de doses unitaires, par exemple de 0,1 myg à 1 mg de produit actif, selon le mode d'administration. Ainsi, par exemple elle peut être de 0,1 myg à 160 myg, de préférence de 1 myg à 80 myg, lorsqu'on administre le composé par voie intraveineuse.

A titre d'exemple non limitatif d'une composition contenant un composé de l'invention, on donne ci-après un exemple d'une solution stérile pour injection intraveineuse:

| | |
|---|---|
| produit A | 250 myg |
| acétate de sodium | 19,15 mg |
| chlorure de sodium | 81 mg |
| acide acétique | 3,59 mg |
| eau pour injection ad | 10 ml. |

Ci-après, on donne des exemples, non limitatifs, de la préparation des 1H-imidazoles conformes à l'invention ainsi que de leurs composés intermédiaires. Dans ces exemples, les spectres de résonance magnétique nucléaire (RMN) ont été relevés avec un appareil Perkin-Elmer à 60 MHz en utilisant le tétraméthylsilane comme référence interne; les déplacements chimiques sont indiqués en delta(ppm). Les lettres s, d, t, q, m et J indiquent respectivement un singulet, doublet, triplet, quartet, multiplet et les constantes de couplage en hertz.

Exemple 1. Préparation des composés de départ de formule II ($R_6$ = H, $R_7$ = alkyle en $C_1$-$C_4$ ou triphénylméthyle) par le procédé a).

A. Obtention des dérivés bromés précurseurs des dérivés organométalliques de formule V.

1. 8-bromo-6-chloro-4H-1,3-benzodioxine.

1.a. 2-bromo-4-chlorophénol.

Ce produit a été préparé selon la méthode de I.I. LAPKIN et al. (voir Zh.Obshch.Khim.35(2),-(1965),251-253; Chem.Abstr.62,(1965), 13111).

1.b. 8-bromo-6-chloro-4H-1,3-benzodioxine.

Dans un mélange de 229 ml d'acide sulfurique concentré et 620 ml d'acide acétique refroidi à 15°C, on ajoute en une fois 311 g de paraformaldéhyde (préalablement lavé avec 200 ml d'acide acétique) et ensuite 462,6 g (2,23 moles) de 2-bromo-4-chlorophénol. On maintient l'agitation pendant 148 heures à 15°C. On neutralise le milieu réactionnel avec 3,1 litres d'une solution aqueuse d'hydroxyde de sodium 7,8N. Le précipité qui s'est formé est filtré, essoré et ensuite dissous dans du toluène. On sèche la solution sur sulfate de sodium, puis on distille. On agite le résidu dans de l'hexane et on laisse cristalliser. On obtient 364 g de 8-bromo-6-chloro-4H-1,3-benzodioxine. Rendement: 65,5%. P.F.: 108-110°C.

| Analyse pour $C_8H_6BrClO_2$ en %: | | | | |
|---|---|---|---|---|
| calculé<br>trouvé | C | 38,47<br>38,9 | H | 2,40<br>2,51 |

2. 6-bromo-4H-1,3-benzodioxine.

Ce produit a été préparé comme indiqué en 1.b. ci-dessus à partir des quantités suivantes de réactifs: 3,8 litres d'acide acétique, 654 ml d'acide sulfurique concentré, 1 kg (5,78 moles) de 4-bromophénol et 870 g (28,9 moles) de paraformaldéhyde. La durée de la réaction est de 120 heures à 0°C.

Après neutralisation du mélange réactionnel par une solution contenant 1350 g d'hydroxyde de sodium dissous dans 13 litres d'eau, le précipité formé est filtré, puis dissous dans 7 litres de toluène. On sèche la phase organique par distillation azéotropique, on filtre à chaud pour éliminer les polymères de formaldéhyde et on évapore sous pression réduite. On distille le résidu sous pression réduite. On obtient 933 g de 6-bromo-4H-1,3-benzodioxine. Rendement: 75%. P.Eb.: 80-90°C/0,13 mbar.

Le produit cristallise par agitation dans un mélange éther isopropylique-hexane. P.F.: 43-47°C.

Spectre RMN ($CDCl_3$): delta 4,88 (2H, s, Ar-$CH_2$), 5,24 (2H, s, -$OCH_2O$-), 6,39 (1H, d, J = 8,7 Hz, ArH), 7,13 (1H, m, ArH), 7,31 (1H, dd, J = 8,7 et 2,4 Hz, ArH).

3. 8-bromo-6-chloro-2,2-diméthyl-4H-1,3-benzodioxine.

3.a. 3-bromo-5-chloro-2-hydroxybenzèneméthanol.

On dissout 880 g (4,241 moles) de 2-bromo-4-chlorophénol dans 8,2 litres d'une solution aqueuse de formaldéhyde à 38% (environ 100 moles). On refroidit au bain de glace, puis on ajoute, par portions de 100 g à la fois, 1.380 g (24 moles) d'hydroxyde de potassium. L'addition se fait en 150 minutes à une température comprise entre 18 et 23°C. On maintient encore l'agitation pendant 2 heures à température ambiante, puis on chauffe progressivement à 40°C au bain-marie. La réaction est légèrement exothermique et la température du milieu se stabilise vers 45°C. On poursuit l'agitation pendant 178 heures à 40°C.

Le milieu réactionnel est ensuite refroidi. On y ajoute 2 litres d'eau et on acidifie jusqu'à pH 3 au moyen de 1420 ml d'acide chlorhydrique concentré. On extrait avec 2 litres de dichlorométhane, puis encore 6 fois par un litre de dichlorométhane. On lave la phase organique avec 2 litres d'eau, on la sèche sur du sulfate de sodium et on évapore le solvant sous pression réduite.

Le résidu brut obtenu (1490 g) contient environ 68,8% de 3-bromo-5-chloro-2-hydroxybenzèneméthanol et environ 23,8% de 2-bromo-4-chlorophénol de départ n'ayant pas réagi.

Ce mélange est utilisé tel quel dans l'étape suivante.

3.b. 8-bromo-6-chloro-2,2-diméthyl-4H-1,3-benzodioxine.

Le résidu brut (1490 g) isolé à l'étape précédente est dissous dans 20 litres de toluène et 4,68 litres de 2,2-diméthoxypropane en présence de 296 g de montmorillonite K10 (fraîchement déshydratée par distillation azéotropique au toluène). La température du mélange s'élève d'elle-même à 30°C; on maintient l'agitation à cette température pendant 115 heures. On filtre le milieu réactionnel et on élimine le toluène sous pression réduite.

On purifie le résidu obtenu par distillation sous pression réduite. On obtient 527 g de 8-bromo-6-chloro-2,2-diméthyl-4H-1,3-benzodioxine pure à 90% (analyse par chromatographie HPLC). P.Eb.: 100-112°C/0,026 mbar. Rendement global: 75% (calculé par rapport au 2-bromo-4-chlorophénol mis en oeuvre).

Spectre RMN ($CDCl_3$): delta 1,55 (6H, s, $C(CH_3)_2$), 4,78 (2H, s, $CH_2$), 6,9 (1H, m, ArH), 7,4 (1H, m, ArH).

4. 6-bromo-2,2-diméthyl-4H-1,3-benzodioxine.

4.a. 5-bromo-2-hydroxybenzèneméthanol.

Ce composé a été préparé comme indiqué en 3.a. ci-dessus à partir de 4-bromophénol.

Après évaporation du dichorométhane ayant servi à l'extraction, on recueille 709 g de résidu qui est chromatographié sur 1,2 kg de silice (éluant: dichlorométhane). Après évaporation du solvant, on obtient 602 g de résidu contenant environ 50% du 5-bromo-2-hydroxybenzèneméthanol souhaité (analyse par chromatographie HPLC).

Ce résidu est utilisé tel quel dans l'étape suivante.

4.b. 6-bromo-2,2-diméthyl-4H-1,3-benzodioxine.

On dissout 572 g du résidu obtenu à l'étape précédente dans 12,5 litres de toluène sec et 2,94 litres de 2,2-diméthoxypropane. On y ajoute 186 g de montmorillonite K10 et on agite le mélange pendant 25 heures à température ambiante.

On filtre, on distille la phase organique sous pression réduite et on redissout le résidu dans 600 ml de toluène. Cette solution est passée au travers d'une colonne contenant 3 kg d'alumine (éluant: 6 litres de toluène). L'éluat est évaporé sous pression réduite et laisse 180 g de résidu qui est distillé sous pression réduite. On obtient 60 g de 6-bromo-2,2-diméthyl-4H-1,3-benzodioxine. P.Eb.: 110-130 °C/0,027 mbar.

Le rendement, calculé par rapport au 4-bromophénol mis en oeuvre, est d'environ 10%.

Spectre RMN ($CDCl_3$): delta 1,52 (6H, s, $C(CH_3)_2$), 4,80 (2H, s, $CH_2$), 6,71 (1H, d, J = 7,9 Hz, ArH), 7,07-7,5 (2H, m, ArH).

5. 8-bromo-2,2,6-triméthyl-4H-1,3-benzodioxine.

Ce composé a été préparé selon la méthode de H.E. KATZ et D.J. CRAM (J.Am.Chem.Soc.106,-(1984),4977). Il est isolé sous la forme d'un résidu qui est utilisé tel quel dans l'étape suivante.

6. 1-bromo-2,6-diméthoxy-3-méthoxyméthylbenzène.

6.a. 3-bromo-2-hydroxy-4-méthoxybenzèneméthanol.

On ajoute goutte à goutte, en 75 minutes et à 0 °C, 28 g (0,12 mole) de 3-bromo-2-hydroxy-4-méthoxybenzoate de méthyle (préparé selon la méthode de T.M. CRESP et al., J.Chem.Soc. Perkin Trans. Partie 1, (1973),340-345) dissous dans 250 ml de tétrahydrofuranne, à une suspension de 6,06 g (0,159 mole) d'hydrure de lithium-aluminium dans 100 ml de tétrahydrofuranne. On agite le mélange pendant trois heures à température ambiante. Ensuite, on ajoute un mélange de 11,5 ml d'eau et 12 ml de tétrahydrofuranne et on acidifie au moyen de 28,6 ml d'acide chlorhydrique concentré dissous dans 300 ml d'eau. On extrait par du dichlorométhane. On lave les phases organiques à l'eau, on les sèche sur du sulfate de sodium et on les évapore sous pression réduite. Le résidu (22,9 g) est utilisé tel quel dans l'étape suivante. Rendement: 91%.

Spectre RMN ($CDCl_3$): delta 3,88 (3H, s, $OCH_3$), 4,72 (2H, s, $CH_2$), 6,45 (1H, d, J = 8,6 Hz, ArH), 7,1 (1H, d, J = 8,6 Hz, ArH).

6.b. 2-bromo-3-méthoxy-6-méthoxyméthylphénol.

On agite à température ambiante pendant 90 minutes un mélange de 22,8 g de 3-bromo-2-hydroxy-4-méthoxybenzèneméthanol, 230 ml de 2,2-diméthoxypropane et 23 g de montmorillonite K10 (fraîchement déshydraté par distillation azéotropique au toluène) dans 200 ml de toluène. Après filtration et élimination du solvant, on obtient 24,1 g de 2-bromo-3-méthoxy-6-méthoxyméthylphénol recristallisable dans l'éther ispropylique.

Rendement: 99%. P.F.: 98-100 °C.

| Analyse pour $C_9H_{11}BrO_3$ en % | | | | |
|---|---|---|---|---|
| calculé<br>trouvé | C | 43,72<br>44,32 | H | 4,45<br>4,49 |

6.c. 1-bromo-2,6-diméthoxy-3-méthoxyméthylbenzène.

On dissout 24,1 g (0,098 mole) de 2-bromo-3-méthoxy-6-méthoxyméthylphénol et 27,7 g d'iodure de méthyle dans 200 ml d'acétone, en présence de 14,82 g (0,107 mole) de carbonate de potassium. On chauffe cette solution à la température de reflux, sous agitation, pendant deux heures et demie. On refroidit, on filtre les sels minéraux et on distille l'acétone. Le résidu est repris par de l'eau et extrait par du dichlorométhane. On sèche la phase organique sur du sulfate de sodium et on la distille sous pression réduite.

On obtient 13,1 g de 1-bromo-2,6-diméthoxy-3-méthoxyméthylbenzène. Rendement: 51%. P.Eb.: 92-115 °C/0,005 mbar.

Spectre RMN ($CDCl_3$): delta 3,4 (3H, s, $OCH_3$), 3,88 (3H, s, $OCH_3$), 3,9 (3H, s, $OCH_3$), 4,48 (2H, s, $CH_2$), 6,74 (1H, s, J = 8,6 Hz, ArH), 7,33 (1H, d, J = 8,6 Hz, ArH).

7. 1-bromo-2,6-diméthoxy-3-(1-méthoxyéthyl)benzène.

7.a. 3-bromo-2-hydroxy-4-méthoxy-alpha-méthylbenzèneméthanol.

On ajoute goutte à goutte, en 80 minutes et à une température comprise entre 13 et 18 °C, 64,3 g (0,262 mole) de 3′-bromo-2′-hydroxy-4′-méthoxyacétophénone (préparée selon la méthode de T.M. CRESP et al. J.Chem.Soc.Perkin Trans. Partie 1,(1973),340-345) en solution dans 560 ml de tétrahydroguranne, à une suspensin de 13,89 g (0,367 mole) de borohydrure de sodium dans 300 ml de tétrahydrofuranne. Ensuite, on maintient l'agitation pendant 2 heures à temperature ambiante. On décompose le mélange réactionnel par addition de 140 ml de tétrahydrofuranne contenant 3 ml d'eau, puis on y ajoute encore 60 ml d'eau. On acidifie le milieu par addition de 415 ml d'une solution aqueuse d'acide chlorhydrique 1N. On élimine le tétrahydrofuranne sous pression réduite et on extrait

la phase aqueuse quatre fois par du dichlorométhane. On sèche les phases organiques sur du sulfate de sodium et on les distille. Le résidu (70 g) est utilisé tel quel dans l'étape suivante.

7.b. 2-bromo-3-méthoxy-6-(1-méthoxyéthyl)phénol.

Ce composé a été préparé comme indiqué en 6.b ci-dessus à partir de 65 g de 3-bromo-2-hydroxy-4-méthoxy-alpha-méthylbenzèneméthanol obtenu à l'étape précédente. Le produit brut obtenu quantitativement est utilisé tel quel dans l'étape suivante.

Spectre RMN ($CDCl_3$): delta 1,48 (3H, d, J = 6,6 Hz, $CH_3$), 3,35 (3H, s, $OCH_3$), 3,88 (3H, s, $OCH_3$), 4,54 (1H, q, J = 6,6 Hz, CH), 6,48 (1H, d, J = 8,6 Hz, ArH), 7,02 (1H, d, J = 8,6 Hz, ArH).

7.c. 1-bromo-2,6-diméthoxy-3-(1-méthoxyéthyl)benzène.

Ce composé est préparé comme indiqué en 6.c. ci-dessus à partir du 2-bromo-3-méthoxy-6-(1-méthoxyéthyl)phénol isolé à l'étape précédente. On obtient 56 g de 1-bromo-2,6-diméthoxy-3-(1-méthoxyéthyl)-benzène.

Rendement: 77%. P.Eb.: 95-120 °C/0,001 mbar.

Spectre RMN ($CDCl_3$): delta 1,42 (3H, d, J = 6,6 Hz, $CH_3$), 3,23 (3H, s, $OCH_3$), 3,88 (3H, s, $OCH_3$), 3,90 (3H, s, $OCH_3$), 4,68 (1H, q, J = 6,6 Hz, CH) 6,79 (1H, d, J = 8,6 Hz, ArH), 7,38 (1H, d, J = 8,6 Hz, ArH).

B. Obtention des 4-($R_3$-CO)-1H-imidazoles de formule VI.

1. 1-triphénylméthyl-1H-imidazole-4-carboxaldéhyde.

1.a. 1-triphénylméthyl-1H-imidazole-4-méthanol.

Ce composé a été préparé selon la méthode décrite par J.L. KELLEY et al. (J.Med.Chem.20,-(1977),721-723).

Rendement: 71,3%. P.F.: 236 °C.

1.b. 1-triphénylméthyl-1H-imidazole-4-carboxaldéhyde.

Ce composé a également été préparé selon la méthode de J.L. KELLEY et al. (loc.cit.) avec un rendement de 87,5%.

P.F.: 190-198 °C.

2. 1-méthyl-1H-imidazole-4-carboxaldéhyde.

Ce produit a été préparé selon le procédé de G. LINDGREN et coll. (J.Het.Chem.17,(1980),679).

3. 5-méthyl-1-triphénylméthyl-1H-imidazole-4-carboxaldéhyde.

3.a. 5-méthyl-1H-imidazole-4-méthanol.

Ce composé a été préparé selon la méthode décrite dans le brevet belge 878.490. Rendement: 59,3%.

3.b. 5-méthyl-1-triphénylméthyl-1H-imidazole-4-méthanol.

A une solution de 100 g (0,673 mole) de 5-méthyl-1H-imidazole-4-méthanol dans 1,5 litre de diméthylformamide maintenue entre 10 et 14 °C, on ajoute en 15 minutes 230 ml (1,659 mole) de triéthylamine. On introduit ensuite, entre 8 et 11 °C, une solution contenant 192 g (0,69 mole) de chlorure de triphénylméthyle dans 2 litres de diméthylformamide.

Le milieu réactionnel est agité pendant 2 heures, puis il est versé sur 14 litres de glace. On continue l'agitation pendant une heure, puis on filtre le précipité, qui est lavé avec 6 litres d'eau et essoré. Ce précipité est ensuite repris par 4 litres d'éthanol à l'ébullition et la fraction non solubilisée est séparée par filtration à chaud. On recueille ainsi un premier jet (19 g; P.F.: 255-260 °C) du produit souhaité. Le filtrat alcoolique est filtré à chaud sur norite puis concentré et refroidi sous agitation. Un deuxième jet du produit souhaité cristallise; on le sépare par filtration: 28,3 g (P.F.: 255-262 °C). On distille finalement le filtrat sous pression réduite et on dissout le résidu dans 1 litre d'un mélange dichlorométhane-méthanol 95:5 et la solution obtenue est purifiée par passage sur une colonne de 1,8 kg de silice (0,2-0,5 mm) (éluant: mélange dichlorométhane-méthanol 80:20). On recueille encore 72,1 g du produit souhaité.

Au total on obtient 119,4 g contenant pratiquement un seul des deux isomères de position 4 et 5 possibles, à savoir le 5-méthyl-1-triphénylméthyl-1H-imidazole-4-méthanol. Rendement: 50,1%.

Le produit obtenu est utilisé tel quel dans l'étape suivante.

3.c. 5-méthyl-1-triphénylméthyl-1H-imidazole-4-carboxaldéhyde.

On chauffe à reflux pendant 85 minutes, en présence de 32,65 g (0,375 mole) de $MnO_2$, une solution de 19 g (0,0536 mole) du produit préparé à l'étape précédente dans 400 ml de chloroforme. On élimine les sels de manganèse par filtration sur dicalite et on distille le filtrat. On recristallise le résidu dans 200 ml d'acétate d'éthyle. On obtient un premier jet de 4,37 g du produit souhaité. Un

second jet de 9,43 g est encore obtenu après concentration des eaux-mères jusqu'à un volume de 60 ml et cristallisation. Rendement: 73,5%.
P.F.: 195-196 ° C.
Le produit obtenu renferme un seul isomère de position.

| Analyse pour $C_{24}H_{20}N_2O$ en %: | | | | | | |
|---|---|---|---|---|---|---|
| calculé<br>trouvé | C | 81,18<br>81,37 | H | 5,68<br>6,25 | N | 7,95<br>7,95 |

4. 1-(1-triphénylméthyl-1H-imidazol-4-yl)-1-éthanone.
4.a. alpha-méthyl-1-triphénylméthyl-1H-imidazole-4-méthanol.
Ce composé a été préparé selon la méthode décrite par J.L. KELLEY et al. (J.Med.Chem.20,-(1977),721-723).
4.b. 1-(1-triphénylméthyl-1H-imidazol-4-yl)-1-éthanone.
On chauffe à reflux pendant 90 minutes un mélange de 221,3 g (2,544 moles) de dioxyde de manganèse et 56,9 g (0,161 mole) de alpha-méthyl-1-triphénylméthyl-1H-imidazole-4-méthanol dissous dans 2,5 litres de chloroforme. On refroidit ensuite la solution vers 50 ° C, on filtre et on élimine le chloroforme par distillation. On dissout le résidu dans 400 ml d'alcool isopropylique et on filtre la solution à chaud sur norite.
Le produit cristallise par refroidissement. On obtient 32,8 g de 1-(1-triphénylméthyl-1H-imidazol-4-yl)-1-éthanone.
Rendement: 58%. P.F.: 158-160 ° C.

| Analyse pour $C_{24}H_{20}N_2O$ en %: | | | | | | |
|---|---|---|---|---|---|---|
| calculé<br>trouvé | C | 81,82<br>81,89 | H | 5,68<br>5,65 | N | 7,95<br>7,90 |

5. 1-(1-triphénylméthyl-1H-imidazol-4-yl)-1-pentanone.
5.a. alpha-n-butyl-1-triphénylméthyl-1H-imidazole-4-méthanol.
Sous atmosphère d'argon, on ajoute lentement une solution de 0,22 mole de bromure de n-butylmagnésium dans 75 ml de tétrahydrofuranne à 67,6 g (0,2 mole) de 1-triphénylméthyl-1H-imidazole-4-carboxaldéhyde (obtenu comme indiqué en 1.b. ci-dessus) dans 500 ml de tétrahydrofuranne. La température du mélange est maintenue aux environs de 20 ° C par refroidissement au bain de glace. Lorsque l'addition est terminée, on agite encore 30 minutes à la température ambiante, puis on ajoute successivement 11 g de chlorure d'ammonium et 100 ml d'eau. On extrait par du dichlorométhane. Les phases organiques sont séchées sur du sulfate de sodium, puis évaporées sous pression réduite. On recristallise le résidu dans un mélange acétate d'éthyle-éther diéthylique 2:1. On obtient 45,8 g de alpha-n-butyl-1-triphénylméthyl-1H-imidazole-4-méthanol.
Rendement: 58%. P.F.: 119-120 ° C.

| Analyse pour $C_{27}H_{28}N_2O$ en %: | | | | | | |
|---|---|---|---|---|---|---|
| calculé<br>trouvé | C | 81,82<br>79,13 | H | 7,07<br>6,82 | N | 7,07<br>6,65 |

5.b. 1-(1-triphénylméthyl-1H-imidazol-4-yl)-1-pentanone.
Ce composé a été préparé comme indiqué en 4.b. ci-dessus à partir du alpha-n-butyl-1-triphénylméthyl-1H-imidazole-4-méthanol obtenu à l'étape précédente.
Le résidu huileux obtenu après évaporation du chloroforme est purifié par chromatographie sur silice (éluant: dichlorométhane). On obtient 39 g de 1-(1-triphénylméthyl-1H-imidazol-4-yl)-1-pentanone.
Rendement: 85,3%. P.F.: 115-118 ° C.

| Analyse pour $C_{27}H_{26}N_2O$ en %: | | | | | | |
|---|---|---|---|---|---|---|
| calculé<br>trouvé | C | 82,23<br>82,18 | H | 6,60<br>6,61 | N | 7,11<br>7,14 |

C. Réaction d'un dérivé organométallique de formule V avec les 4-($R_3$-CO)-1H-imidazoles de formule VI.

1. alpha-(6-chloro-4H-1,3-benzodioxin-8-yl)-1-triphénylméthyl-1H-imidazole-4-méthanol.

Sous atmosphère d'azote, on ajoute goutte à goutte 121 g (0,485 mole) de 8-bromo-6-chloro-4H-1,3-benzodioxine, dissous dans 400 ml de tétrahydrofuranne sec, à une suspension de 12,16 g (0,5 mole) de magnésium dans 430 ml de tétrahydrofuranne anhydre porté à reflux.

Lorsque l'addition est terminée, on maintient encore le reflux pendant une demi-heure, puis on refroidit vers 40 °C. On ajoute le composé organomagnésien ainsi formé rapidement à 164 g (0,485 mole) de 1-triphénylméthyl-1H-imidazole-4-carboxaldéhyde en solution dans 2 litres de tétrahydrofuranne préchauffé à 40 °C.

La température s'élève d'elle-même à 50 °C au cours de l'addition. On maintient l'agitation pendant une heure à 40 °C. On refroidit ensuite le mélange réactionnel à 0 °C et on le décompose par addition d'un litre d'une solution saturée de chlorure d'ammonium. On filtre le précipité formé et on le lave avec du méthanol et de l'éther. On recueille 140,7 g de produit. Ce produit est encore purifié par agitation dans un litre d'eau. On filtre et lave à l'éthanol, puis à l'éther diéthylique. On obtient 121,3 g de alpha-(6-chloro-4H-1,3-benzodioxin-8-yl)-1-triphénylméthyl-1H-imidazole-4-méthanol.
Rendement: 49,2%. P.F.: 233-235 °C.

| Analyse pour $C_{31}H_{25}ClN_2O_3$ en %: | | | | | | |
|---|---|---|---|---|---|---|
| calculé<br>trouvé | C | 73,15<br>73,21 | H | 4,91<br>4,94 | N | 5,50<br>5,48 |

2. alpha-(4H-1,3-benzodioxin-6-yl)-1-triphénylméthyl-1H-imidazole-4-méthanol.

Ce composé a été préparé comme le composé précédent mais à partir de 6-bromo-4H-1,3-benzodioxine et de 1-triphénylméthyl-1H-imidazole-4-carboxaldéhyde. Après décomposition du milieu réactionnel, on extrait le produit de la réaction au dichlorométhane et on le recristallise dans l'alcool isopropylique. Par chromatographie du résidu obtenu après évaporation des eaux-mères, on récupère encore un second jet de produit.
Rendement: 53%. P.F.: 165-167 °C.

| Analyse pour $C_{31}H_{26}N_2O_3$ en %: | | | | | | |
|---|---|---|---|---|---|---|
| calculé<br>trouvé | C | 78,48<br>78,19 | H | 5,48<br>5,82 | N | 5,91<br>5,84 |

3. alpha-(6-chloro-2,2-diméthyl-4H-1,3-benzodioxin-8-yl)-1-triphénylméthyl-1H-imidazole-4-méthanol.

A une suspension de 26,73 g (1 mole + 10% d'excès) de magnésium dans 250 ml de tétrahydrofuranne anhydre, on ajoute 2 ml de dibromoéthane et on chauffe vers 30 °C pour déclencher la réaction. On ajoute de suite goutte à goutte 277,5 g (1 mole) de 8-bromo-6-chloro-2,2-diméthyl-4H-1,3-benzodioxine dissous dans 250 ml de tétrahydrofuranne de manière à ce que la température du milieu ne dépasse pas 40 °C.

L'addition dure environ 150 minutes. On refroidit le composé organomagnésien vers 10 °C (précipitation partielle) et on l'ajoute à une solution de 338 g (1 mole) de 1-triphénylméthyl-1H-imidazole-4-carboxaldéhyde dans 2,8 litres de tétrahydrofuranne, préalablement refroidie à 0 °C. Pendant l'addition, la température du mélange s'élève progressivement jusqu'à 20 °C. On poursuit l'agitation encore pendant une heure à cette température, puis on ajoute 53,5 g (1 mole) de chlorure d'ammonium.

On agite pendant 1 heure, on ajoute encore 18 ml d'eau et on maintient l'agitation pendant une heure supplémentaire.

On élimine le tétrahydrofuranne sous pression réduite. On reprend le résidu dans 5 litres de dichlorométhane et on lave avec 2 litres d'eau contenant 30 g de bisulfite de sodium. On sépare la

phase aqueuse et on la lave avec 1 litre de dichlorométhane. Les phases organiques sont encore lavées à l'eau puis séchées sur du sulfate de sodium et on élimine le solvant sous pression réduite. Le résidu est recristallisé dans environ 4 litres de toluène à 80 °C et filtré à chaud sur norite. On obtient ainsi 335,6 g de alpha-(6-chloro-2,2-diméthyl-4H-1,3-benzodioxin-8-yl)-1-triphénylméthyl-1H-imidazole-4-méthanol qui contient une molécule de toluène.
P.F.: 120 °C suivi de 188 °C.

| Analyse pour $C_{33}H_{29}ClN_2O_3$ + $C_7H_8$ en %: | | | | | | |
|---|---|---|---|---|---|---|
| calculé<br>trouvé | C | 76,81<br>74,78 | H | 5,78<br>5,24 | N | 4,37<br>4,73 |

On a préparé les composés suivants par la méthode décrite au point 3. ci-dessus.

4. alpha-(6-chloro-2,2-diméthyl-4H-1,3-benzodioxin-8-yl)-1-méthyl-1H-imidazole-4-méthanol.

A partir de 8-bromo-6-chloro-2,2-diméthyl-4H-1,3-benzodioxine et de 1-méthyl-1H-imidazole-4-carboxaldéhyde. L'addition du composé organomagnésien est effectuée à 0 °C.
Rendement: 63,5%. P.F.: 131-136 °C (acétate d'éthyle).

| Analyse pour $C_{17}H_{17}ClN_2O_3$ en %: | | | | | | | | |
|---|---|---|---|---|---|---|---|---|
| calculé<br>trouvé | C | 58,35<br>58,47 | H | 5,51<br>5,54 | N | 9,08<br>8,97 | Cl | 11,55<br>11,49 |

5. alpha-(6-chloro-2,2-diméthyl-4H-1,3-benzodioxin-8-yl)-5-méthyl-1-triphénylméthyl-1H-imidazole-4-méthanol.

A partir de 8-bromo-6-chloro-2,2-diméthyl-4H-1,3-benzodioxine- et de 5 méthyl-1-triphénylméthyl-1H-imidazole-4-carboxaldéhyde.

L'addition du composé organomagnésien s'effectue à 0 °C.
Rendement: 50%. P.F.: 100-120 °C (acétonitrile).

Spectre RMN ($CDCl_3$): delta 1,4 (6H, m, $CH_3$-C-$CH_3$), 1,94 (3H, s, $CH_3$), 4,79 (2H, s, $CH_2$), 5,98 (1H, s, CHOH), 6,65-7,70 (18H, m, ArH + ImH).

6. alpha-(2,2,6-triméthyl-4H-1,3-benzodioxin-8-yl)-1-triphénylméthyl-1H-imidazole-4-méthanol.

A partir de 8-bromo-2,2,6-triméthyl-4H-1,3-benzodioxine et de 1-triphénylméthyl-1H-imidazole-4-carboxaldéhyde. Le composé organomagnésien est ajouté à la température ambiante.

Le produit de réaction est purifié par chromatographie sur silice (15 mym) (éluant: dichlorométhane-méthanol 98:2).
Rendement: 31%. P.F.: 205-215 °C (acétonitrile).

Spectre RMN ($CDCl_3$): delta 1,3 (3H, s, $CH_3$-C-$CH_3$), 1,38 (3H, s, $CH_3$-C-$CH_3$), 2,21 (3H, s, $CH_3$), 4,78 (2H, s, $CH_2$), 6,02 (1H, large s, CHOH), 6,72 (2H, m, ImH + OH), 7,0-7,65 (18H, m, ArH + ImH).

7. alpha-(2,2-diméthyl-4H-1,3-benzodioxin-6-yl)-1-triphénylméthyl-1H-imidazole-4-méthanol.

A partir de 6-bromo-2,2-diméthyl-4H-1,3-benzodioxine et de 1-triphénylméthyl-1H-imidazole-4-carboxaldéhyde. La témperature du milieu réactionnel ne dépasse pas 40 °C.
Rendement: 54,5%. P.F.: 155-162 °C (acétonitrile).

Spectre RMN (DMSO): delta 1,43 (6H, s, $CH_3$-C-$CH_3$), 4,77 (2H, s, $CH_2$), 5,53 (2H, s, CH et OH) 6,6-7,7 (20 H, m, ArH + ImH).

8. alpha-(2,6-diméthoxy-3-méthoxyméthylphényl)-1-triphénylméthyl-1H-imidazole-4-méthanol.

A partir de 1-bromo-2,6-diméthoxy-3-méthoxyméthylbenzène et de 1-triphénylméthyl-1H-imidazole-4-carboxaldéhyde. Le produit de réaction est purifié par chromatographie sur silice et se présente sous la forme d'une laque vitreuse.
Rendement: 48%.

Spectre RMN ($CDCl_3$): delta 3,36 (3H, s, $OCH_3$), 3,7 (6H, s, 2 × $OCH_3$), 4,43 (2H, s, $CH_2$), 6,0-6,4 (1H, m, CH), 6,5-7,6 (20 H, m, ArH + ImH + OH).

9. alpha-[2,6-diméthoxy-3-(1-méthoxyéthyl)phényl]-1-triphénylméthyl-1H-imidazole-4-méthanol.

A partir de 1-bromo-2,6-diméthoxy-3-(1-méthoxyéthyl)benzène et de 1-triphénylméthyl-1H-imidazole-4-carboxaldéhyde. Le produit de réaction est purifié par chromatographie sur silice (éluant: mélange dichlorométhane-méthanol 98:2).

Rendement: 49,7%. P.F.: 96-99 ° C (acétonitrile).

| Analyse pour $C_{34}H_{34}N_2O_4$ en %: | | | | | | |
|---|---|---|---|---|---|---|
| calculé<br>trouvé | C | 76,4<br>76,37 | H | 6,36<br>6,31 | N | 5,24<br>5,29 |

10. alpha-(6-chloro-2,2-diméthyl-4H-1,3-benzodioxin-8-yl)-alpha-méthyl-1-triphénylméthyl-1H-imidazole-4-méthanol.

On prépare le composé organomagnésien de la 8-bromo-6-chloro-2,2-diméthyl-4H-1,3-benzodioxine comme indiqué en 3. ci-dessus. Puis, en 20 minutes et à une température ne dépassant pas 25 ° C, on ajoute 15 g (0,046 mole) de ce composé organomagnésien à 15 g (0,0426 mole) de 1-(1-triphénylméthyl-1H-imidazol-4-yl)-1-éthanone en solution dans 150 ml de tétrahydrofuranne. On agite pendant 165 minutes, puis on décompose le milieu réactionnel par 2,5 g de chlorure d'ammonium dissous dans 50 ml d'eau. On extrait par du dichlorométhane. La phase organique est séchée sur du sulfate de sodium puis distillée. Le résidu est purifié par chromatographie sur silice (éluant: mélange dichlorométhane-méthanol 98:2). On obtient 5,68 g de alpha-(6-chloro-2,2-diméthyl-4H-1,3-benzodioxin-8-yl)-alpha-méthyl-1-triphénylméthyl-1H-imidazole-4-méthanol.

P.F.: 182-184 ° C (acétate d'éthyle). Ce composé est identique à celui préparé à l'exemple 2.B.2.

11. alpha-n-butyl-alpha-(6-chloro-2,2-diméthyl-4H-1,3-benzodioxin-8-yl)-1-triphénylméthyl-1H-imidazole-4-méthanol.

Ce composé a été préparé comme le composé précédent en faisant réagir le composé organomagnésien de la 8-bromo-6-chloro-2,2-diméthyl-4H-1,3-benzodioxine avec la 1-(1-triphénylméthyl-1H-imidazol-4-yl)-1-pentanone.

Rendement: 54,6%. P.F.: 124 ° C (éther de pétrole).

Spectre RMN ($CDCl_3$): delta 0,6-3,3 (15H, m, $C_4H_9$ et $CH_3$-C-$CH_3$), 4,26 (1H, large s, OH), 4,78 (2H, s, $CH_2$), 6,7-8,0 (19H, m, ArH + ImH).

12. alpha-(2,2-diméthyl-4H-1,3-benzodioxin-8-yl)-1-méthyl-1H-imidazole-4-méthanol (chlorhydrate).

On hydrogénolyse 15,88 g d'alpha-(6-chloro-2,2-diméthyl-4H-1,3-benzodioxin-8-yl)-1-méthyl-1H-imidazole-4-méthanol (préparé au point 4. ci-dessus) dissous dans 160 ml de méthanol en présence de 3 g de charbon palladié à 10%, sous une pression d'hydrogène de 3,5 bars à 50 ° C pendant 150 minutes. Puis, on filtre le catalyseur, on élimine le solvant et le résidu est agité dans de l'éther diéthylique. La phase éthérée est décantée et le résidu obtenu est utilisé tel quel dans l'étape suivante.

Exemple 2. Préparation des composés de départ de formule II ($R_6$ = H, $R_7$ = alkyle en $C_1$-$C_4$ ou triphénylméthyle) par le procédé b).

A. Obtention des cétones de formule VII.

1. (6-chloro-4H-1,3-benzodioxin-8-yl) (1-triphénylméthyl-1H-imidazol-4-yl)cétone.

Ce composé a été préparé selon le procédé décrit à l'exemple 1.B.4.b. à partir d'alpha-(6-chloro-4H-1,3-benzodioxin-8-yl)-1-triphénylméthyl-1H-imidazole-4-méthanol (préparé à l'exemple 1.C.1).

Rendement: 95%. P.F.: 175-182 ° C. Un échantillon recristallisé dans l'éthanol fond à 182-185 ° C et 203 ° C.

| Analyse pour $C_{31}H_{23}ClN_2O_3$ en %: | | | | | | | | |
|---|---|---|---|---|---|---|---|---|
| calculé<br>trouvé | C | 73,45<br>72,48 | H | 4,54<br>4,48 | N | 5,53<br>5,18 | Cl | 7,01<br>6,96 |

2. (6-chloro-2,2-diméthyl-4H-1,3-benzodioxin-8-yl) (1-triphénylméthyl-1H-imidazol-4-yl)cétone.

Ce composé a été préparé selon le procédé décrit à l'exemple 1.B.4.b. à partir d'alpha-(6-chloro-2,2-diméthyl-4H-1,3-benzodioxin-8-yl)-1-triphénylméthyl-1H-imidazole-4-méthanol (préparé à l'exemple 1.C.3).

Rendement: 88% (produit pratiquement pur). P.F.: 200-205 ° C.

| Analyse pour $C_{33}H_{27}ClN_2O_3$ en %: | | | | | | | | |
|---|---|---|---|---|---|---|---|---|
| calculé<br>trouvé | C | 74,08<br>74,17 | H | 5,05<br>5,03 | N | 5,24<br>5,22 | Cl | 6,64<br>6,73 |

B. Réaction d'une cétone de formule VII avec un composé organomagnésien de formule VIII.

1. alpha-(6-chloro-4H-1,3-benzodioxin-8-yl)-alpha-méthyl-1-triphénylméthyl-1H-imidazole-4-méthanol.

On ajoute une suspension de 0,148 mole d'iodure de méthylmagnésium dans 150 ml d'éther diéthylique à 30 °C, à la température ambiante, à 17,3 g (0,033 mole) de (6-chloro-4H-1,3-benzodioxin-8-yl) (1-triphénylméthyl-1H-imidazol-4-yl)cétone en solution dans 200 ml de tétrahydrofuranne. La température du milieu réactionnel s'élève jusqu'à 40 °C. Lorsque l'addition est terminée, on maintient l'agitation pendant une heure à température ambiante. On ajoute 8 g de chlorure d'ammonium et on maintient encore l'agitation pendant une heure. On ajoute ensuite 100 ml d'eau et on extrait le milieu réactionnel deux fois par du dichlorométhane. On sèche les phases organiques sur du sulfate de sodium et on élimine le solvant sous pression réduite. Le résidu est recristallisé dans 100 ml d'acétate d'éthyle. On obtient 13,77 g d'alpha-(6-chloro-4H-1,3-benzodioxin-8-yl)-alpha-méthyl-1-triphénylméthyl-1H-imidazole-4-méthanol.

Rendement: 80%. P.F.: 248-250 °C (décomposition).

| Analyse pour $C_{32}H_{27}ClN_2O_3$ en %: | | | | | | | | |
|---|---|---|---|---|---|---|---|---|
| calculé<br>trouvé | C | 73,49<br>73,41 | H | 5,16<br>5,05 | N | 5,36<br>5,26 | Cl | 6,7<br>6,93 |

2. alpha-(6-chloro-2,2-diméthyl-4H-1,3-benzodioxin-8-yl)-alpha-méthyl-1-triphénylméthyl-1H-imidazole-4-méthanol.

Ce composé a été préparé comme le composé précédent à partir de l'iodure de méthylmagnésium et de (6-chloro-2,2-diméthyl-4H-1,3-benzodioxin-8-yl) (1-triphénylméthyl-1H-imidazol-4-yl)cétone. Le résidu obtenu après évaporation du dichlorométhane cristallise sous agitation dans de l'éther diéthylique.

Rendement: 80%. P.F.: 182-184 °C (acétate d'éthyle).

| Analyse pour $C_{34}H_{31}ClN_2O_3$ en %: | | | | | | | | |
|---|---|---|---|---|---|---|---|---|
| calculé<br>trouvé | C | 74,11<br>73,98 | H | 5,63<br>5,65 | N | 5,08<br>5,00 | Cl | 6,45<br>6,49 |

Ce composé est identique à celui préparé à l'exemple 1.C.10.

Exemple 3. Préparation des composés de départ de formule II ($R_6$ = H ou alkyle en $C_1$-$C_4$, $R_7$ = H).

1. 4[(2,2-diméthyl-4H-1,3-benzodioxin-8-yl)(méthoxy)méthyl]-1H-imidazole (chlorhydrate) et alpha-(2,2-diméthyl-4H-1,3-benzodioxin-8-yl)-1H-imidazole-4-méthanol (chlorhydrate).

On hydrogénolyse 125,4 g (0,23 mole) d'alpha-(6-chloro-2,2-diméthyl-4H-1,3-benzodioxin-8-yl)-1-triphénylméthyl-1H-imidazol-4-méthanol (préparé à l'exemple 1.C.3.) partiellement dissous dans 1250 ml de méthanol, en présence de 6 g de charbon palladié à 10% et sous une pression d'hydrogène initiale de 2,7 bars.

La réaction est effectuée à une température de 60 °C. Puis, on filtre le catalyseur sur hyflo-cel et on élimine le méthanol sous pression réduite. On reprend le résidu dans 100 ml de méthanol et on refroidit dans un bain de glace. On élimine par filtration le triphénylméthane qui a cristallisé et on évapore ensuite le filtrat. Le résidu obtenu est agité pendant au moins 12 heures dans 650 ml d'éther diéthylique. On filtre le précipité et on lave à l'éther diéthylique. On obtient 64,59 g d'une poudre amorphe qui est formée d'un mélange de l'alcool souhaité et du dérivé O-méthylé dans la proportion 35/65 déterminée par RMN. Ce mélange est utilisé tel quel dans l'étape suivante. Un échantillon de 5,36 g de ce mélange de produits est recristallisé dans 20 ml d'un mélange éthanol-éther 1:1. On isole 1,35 g du chlorhydrate d'alpha-(2,2-diméthyl-4H-1,3-benzodioxin-8-yl)-1H-imidazole-4-méthanol pur. Ce produit n'a pas de point

de fusion net (décomposition).

| Analyse pour $C_{14}H_{16}N_2O_3$ . HCl en %: | | | | | | | | |
|---|---|---|---|---|---|---|---|---|
| calculé<br>trouvé | C | 56,66<br>55,81 | H | 5,40<br>5,77 | N | 9,44<br>8,94 | $Cl^-$ | 11,97<br>11,83 |

Le dérivé O-méthylé qui se forme au cours de l'hydrogénolyse a été séparé du milieu réactionnel, neutralisé par addition d'ammoniac et en même temps purifié par chromatographie. Le 4-[(2,2-diméthyl-4H-1,3,-benzodioxin-8-yl)(méthoxy)méthyl]-1H-imidazole obtenu est transformé en chlorhydrate par addition d'une solution d'acide chlorhydrique dans le méthanol. P.F.: 150-155 °C (décomposition).

| Analyse pour $C_{15}H_{18}N_2O_3$ . HCl en %: | | | | | | | | |
|---|---|---|---|---|---|---|---|---|
| calculé<br>trouvé | C | 57,97<br>58,12 | H | 6,12<br>6,08 | N | 9,02<br>9,10 | $Cl^-$ | 11,43<br>11,40 |

2. alpha-(2,2-diméthyl-4H-1,3-benzodioxin-8-yl)-5-méthyl-1H-imidazole-4-méthanol (chlorhydrate) et 4-[-(2,2-diméthyl-4H-1,3-benzodioxin-8-yl)(méthoxy)méthyl]-5-méthyl-1H-imidazole (chlorhydrate).

On hydrogénolyse 17,5 g (0,032 mole) d'alpha-(6-chloro-2,2-diméthyl-4H-1,3-benzodioxin-8-yl)-5-méthyl-1-triphénylméthyl-1H-imidazole-4-méthanol (préparé à l'exemple 1.C.5.) dissous dans 300 ml de méthanol en présence de 1,5 g de charbon palladié à 10%, sous une pression d'hydrogène de 3,5 bars à 50 °C pendant 3 heures. Puis on filtre le catalyseur, on élimine le solvant et le résidu est agité dans de l'éther diéthylique pour éliminer le triphénylméthane. Le résidu obtenu après décantation de la phase éthérée est caractérisé par son spectre RMN et est un mélange comprenant 65% du chlorhydrate du dérivé O-méthylé et 35% du chlorhydrate de l'alcool. Ce mélange est utilisé tel quel dans l'étape suivante.

3. 4-[(2,2,6-triméthyl-4H-1,3-benzodioxin-8-yl) (méthoxy)méthyl]-1H-imidazole et 4-[(2,2,6-triméthyl-4H-1,3-benzodioxin-8-yl)méthyl]-1H-imidazole.

On hydrogénolyse 9,45 g (0,0183 mole) d'alpha-(2,2,6-triméthyl-4H-1,3-benzodioxin-8-yl)-1-triphénylméthyl-1H-imidazole-4-méthanol (préparé à l'exemple 1.C.6.) dissous dans 300 ml de méthanol, en présence de 0,6 g de charbon palladié à 10%, pendant 4 heures à 80 °C sous une pression d'hydrogène de 2 bars. Puis, on filtre le catalyseur et on élimine le solvant. Le résidu obtenu est agité dans de l'éther diéthylique pour éliminer le triphénylméthane, puis chromatographié sur 700 g de silice (10 mym) (éluant: dichlorométhane-méthanol 95:5). On isole un mélange 40:60 de 4-[(2,2,6-triméthyl-4H-1,3-benzodioxin-8-yl)méthyl]-1H-imidazole et de 4-[(2,2,6-triméthyl-4H-1,3-benzodioxin-8-yl)(méthoxy)-méthyl]-1H-imidazole, identifié par RMN par la présence du pic correspondant au radical méthoxy (DMSO: delta 3,13). Ce mélange est utilisé tel quel dans l'étape suivante.

4. alpha-(2,2-diméthyl-4H-1,3-benzodioxin-6-yl)-1H-imidazole-4-méthanol.

Le procédé utilisé est le même que celui décrit au point 2. ci-dessus, mais on part de l'alpha-(2,2-diméthyl-4H-1,3-benzodioxin-6-yl)-1-triphénylméthyl-1H-imidazole-4-méthanol (préparé à l'exemple 1.C.7.). Le résidu obtenu est chromatographié sur silice (15 mym) (éluant: dichlorométhane-méthanol 95:5). L'alpha-(2,2-diméthyl-4H-1,3-benzodioxin-6-yl)-1H-imidazole-4-méthanol isolé est recristallisé dans l'acétate d'éthyle. P.F.: 96 °C.

| Analyse pour $C_{14}H_{16}N_2O_3$ en %: | | | | | | |
|---|---|---|---|---|---|---|
| calculé<br>trouvé | C | 64,62<br>64,37 | H | 6,15<br>6,40 | N | 10,77<br>10,68 |

5. alpha-(2,2-diméthyl-4H-1,3-benzodioxin-8-yl)-1H-imidazole-4-méthanol.

A une suspension de 21,46 g (0,04 mole) d'alpha-(6-chloro-2,2-diméthyl-4H-1,3-benzodioxin-8-yl)-1-triphénylméthyl-1H-imidazole-4-méthanol (préparé à l'exemple 1.C.3.) dans 2 litres d'ammoniac et 200 ml de toluène, on ajoute par morceaux 5,75 g (0,25 mole) de sodium. On maintient l'agitation pendant 40 minutes, puis on décompose le milieu réactionnel par addition de 6,42 g (0,12 mole) de chlorure d'ammonium. On ajoute 500 ml de toluène contenant 10% de méthanol, on chasse l'ammoniac et on ajoute encore 500 ml d'eau. On décante la phase toluénique, on sèche sur du sulfate de sodium, puis on

distille. Le résidu qui contient du triphénylméthane est partiellement dissous dans 50 ml d'eau contenant 3,3 ml d'acide chlorhydrique concentré et la suspension obtenue est extraite par de l'éther. La phase aqueuse est ensuite amenée à pH 8 par addition de bicarbonate de sodium, puis extraite par du dichlorométhane. Ensuite, on élimine le solvant par distillation et on obtient 3,5 g d'alpha-(2,2-diméthyl-4H-1,3-benzodioxin-8-yl)-1H-imidazole-4-méthanol.
Rendement: 33%.

Le produit forme un chlorhydrate (recristallisable dans un mélange éthanol-éther 1:1) qui se présente sous un aspect vitreux et n'a pas de point de fusion net.

| Analyse pour $C_{14}H_{16}N_2O_3$ . HCl en %: | | | | | | | | |
|---|---|---|---|---|---|---|---|---|
| calculé<br>trouvé | C | 56,66<br>55,81 | H | 5,40<br>5,77 | N | 9,44<br>8,94 | $Cl^-$ | 11,97<br>11,83 |

6. alpha-(2,6-diméthoxy-3-méthoxyméthylphényl)-1H-imidazole-4-méthanol.

On procède comme au point 1. ci-dessus, mais à 80°C au départ de l'alpha-(2,6-diméthoxy-3-méthoxyméthylphényl)-1-triphénylméthyl-1H-imidazole-4-méthanol (préparé à l'exemple 1.C.8.). Après élimination du triphénylméthane cristallisé et évaporation du méthanol, le résidu obtenu est purifié par chromatographie sur silice (éluant: mélange dichlorométhane-méthanol 80:20). On obtient une huile caractérisée par son spectre de résonance nucléaire magnétique.
Rendement: 68%.

Spectre RMN ($CDCl_3$): delta 3,38 (3H, s, $OCH_3$), 3,66 (3H, s, $OCH_3$), 3,76 (3H, s, $OCH_3$), 4,43 (2H, s, $CH_2$), 6,28 (1H, s, CH), 6,69-7,70 (6H, m, ArH + ImH + OH + NH).

7. alpha-[(2,6-diméthoxy-3-(1-méthoxyéthyl)phényl]-1H-imidazole-4-méthanol.

On procède comme au point 6. ci-dessus par hydrogénolyse d'alpha-[(2,6-diméthoxy-3-(1-méthoxyéthyl)phényl]-1-triphénylméthyl-1H-imidazole-4-méthanol (préparé à l'exemple 1.C.9.).
Rendement: 79,6%. P.F.: 138-145°C (tétrahydrofuranne-éther).

| Analyse pour $C_{15}H_{20}N_2O_4$ en %: | | | | | | |
|---|---|---|---|---|---|---|
| calculé<br>trouvé | C | 61,64<br>61,48 | H | 6,85<br>7,0 | N | 9,59<br>9,38 |

8. alpha-n-butyl-alpha-(2,2-diméthyl-4H-1,3-benzodioxin-8-yl)-1H-imidazole-4-méthanol.

On procède comme au point 1. ci-dessus, mais à 80°C au départ de l'alpha-n-butyl-alpha-(6-chloro-2,2-diméthyl-4H-1,3-benzodioxin-8-yl)-1-triphénylméthyl-1H-imidazole-4-méthanol (préparé à l'exemple 1.C.11). Le résidu obtenu après filtration du triphénylméthane et évaporation du méthanol est utilisé tel quel dans l'étape suivante.

9. chlorhydrate d'alpha-(2,2-diméthyl-4H-1,3-benzodioxin-8-yl)-alpha-méthyl-1H-imidazole-4-méthanol.

On procède comme au point 2. ci-dessus, mais à 20°C au départ de l'alpha-(6-chloro-2,2-diméthyl-4H-1,3-benzodioxin-8-yl)-alpha-méthyl-1-triphénylméthyl-1H-imidazole-4-méthanol (préparé à l'exemple 2.B.2.). Rendement presque quantitatif. P.F.: 85-100°C. (décomposition).

Un échantillon analytique est préparé par agitation dans de l'éther diéthylique. P.F.: 72-90°C (décomposition).

| Analyse pour $C_{15}H_{18}N_2O_3$ . HCl en %: | | | | | | | | |
|---|---|---|---|---|---|---|---|---|
| calculé<br>trouvé | C | 57,97<br>57,94 | H | 6,12<br>6,95 | N | 9,01<br>8,12 | $Cl^-$ | 11,43<br>9,51 |

Exemple 4. Préparation des 1H-imidazoles de formule I ($Z_1 = Z_2$ = alkyle et $Z_1 + Z_2 = -CH_2-$).

1. chlorhydrate de 4-[(4H-1,3-benzodioxin-8-yl)méthyl]-1H-imidazole.

On hydrogénolyse 50,85 g (0,1 mole) d'alpha-(6-chloro-4H-1,3-benzodioxin-8-yl)-1-triphénylméthyl-1H-imidazole-4-méthanol (préparé à l'exemple 1.C.1.) dissous dans 500 ml d'acide acétique, en présence de 3 g de charbon palladié à 10% pendant 2 heures à 80°C sous une pression d'hydrogène de 2,41

bars. Puis, on élimine le catalyseur par filtration et on distille le solvant sous pression réduite. Le résidu obtenu est extrait trois fois par de l'éther diéthylique pour éliminer le triphénylméthane qui s'est formé. On le recristallise ensuite dans 50 ml d'acétonitrile. On obtient 21,2 g de chlorhydrate de 4-[(4H-1,3-benzodioxin-8-yl)méthyl]-1H-imidazole.
Rendement: 83%. P.F.: 168-173 °C.

| Analyse pour $C_{12}H_{12}N_2O_2$ . HCl en %: | | | | | | | | |
|---|---|---|---|---|---|---|---|---|
| calculé<br>trouvé | C | 57,03<br>57,01 | H | 5,15<br>5,20 | N | 11,09<br>11,02 | $Cl^-$ | 14,06<br>13,83 |

2. 4-[(4H-1,3-benzodioxin-6-yl)méthyl]-1H-imidazole.

Ce composé a été préparé comme le composé précédent, mais à partir de 25 g (0,052 mole) d'alpha-(4H-1,3-benzodioxin-6-yl)-1-triphénylméthyl-1H-imidazole-4-méthanol (préparé à l'exemple 1.C.2.). Le triphénylméthane formé est extrait plusieurs fois par de l'hexane et le résidu est purifié par chromatographie sur silice (éluant: dichlorométhane-méthanol 95:5). Les fractions contenant le produit sont évaporées et le résidu est repris par une solution diluée d'ammoniac dans le méthanol. On distille cette solution et on recristallise le résidu dans l'acétate d'éthyle.

On obtient 3,5 g de 4-[(4H-1,3-benzodioxin-6-yl)méthyl]-1H-imidazole.
Rendement: 31,2%. P.F.: 145 °C.

| Analyse pour $C_{12}H_{12}N_2O_2$ en %: | | | | | | |
|---|---|---|---|---|---|---|
| calculé<br>trouvé | C | 66,66<br>66,14 | H | 5,55<br>5,62 | N | 12,96<br>12,69 |

3. 4-[(2,6-diméthoxy-3-méthoxyméthylphényl)méthyl]-1H-imidazole (chlorhydrate).

Dans un litre d'ammoniac liquide, on introduit 6,75 g (0,0243 mole) d'alpha-(2,6-diméthoxy-3-méthoxyméthylphényl)-1H-imidazole-4-méthanol (préparé à l'exemple 3.6.) préalablement dissous dans 200 ml de tétrahydrofuranne. On ajoute en premier lieu 1,3 g (0,0243 mole) de chlorure d'ammonium et ensuite, en 30 minutes, 1,12 g (0,0486 mole) de sodium par morceaux. Après la disparition complète du sodium, on ajoute 1,3 g de chlorure d'ammonium et on maintient l'agitation du milieu réactionnel pendant 30 minutes. On ajoute 300 ml de toluène contenant 10 ml de méthanol. L'ammoniac est ensuite chassé au bain-marie. On ajoute encore 100 ml d'eau et l'on décante la phase organique. Celle-ci est séchée sur du sulfate de sodium et le solvant est évaporé sous pression réduite.

Le produit obtenu est purifié par chromatographie sur silice (éluant: mélange dichlorométhane-méthanol-ammoniaque 95:4,5:0,5). On obtient 1,81 g sous forme d'une laque (rendement: 28,4%). Il est dissous dans de l'éther isopropylique et transformé en chlorhydrate par addition d'un équivalent d'une solution d'acide chlorhydrique dans de l'alcool isopropylique. P.F.: 130-135 °C.

| Analyse pour $C_{14}H_{18}N_2O_3$ . HCl en %: | | | | | | |
|---|---|---|---|---|---|---|
| calculé<br>trouvé | C | 56,28<br>56,13 | H | 6,36<br>6,39 | N | 9,38<br>9,10 |

4. 4-[[2,6-diméthoxy-3-(1-méthoxyéthyl)phényl]méthyl]-1H-imidazole (chlorhydrate).

On procède comme au point 3. ci-dessus, mais au départ de l'alpha-[2,6-diméthoxy-3-(1-méthoxyéthyl)phényl]-1H-imidazole-4-méthanol (préparé à l'exemple 3.7.). L'huile obtenue après évaporation du toluène est reprise par de l'éther et transformée en chlorhydrate par addition d'une solution d'acide chlorhydrique dans du méthanol.
Rendement: 68,5%. P.F.: 168-170 °C.

| Analyse pour $C_{15}H_{20}N_2O_3$ . HCl en %: | | | | | | | | |
|---|---|---|---|---|---|---|---|---|
| calculé<br>trouvé | C | 57,6<br>57,52 | H | 6,72<br>6,84 | N | 8,96<br>8,89 | $Cl^-$ | 11,36<br>11,27 |

5. 4-[1-(4H-1,3-benzodioxin-8-yl)éthyl]-1H-imidazole.

On procède comme au point 1. ci-dessus, mais au départ de l'alpha-(6-chloro-4H-1,3-benzodioxin-8-yl)-alpha-méthyl-1-triphénylméthyl-1H-imidazole-4-méthanol (préparé à l'exemple 2.B.1.). Après élimination du triphénylméthane, le résidu obtenu est dissous dans de l'eau et la solution est alcalinisée à pH 8 par addition d'une solution saturée de carbonate de sodium. Le précipité qui se forme est redissous dans du dichlorométhane. On sèche la phase organique sur du sulfate de sodium et on évapore le solvant sous pression réduite. Le résidu est chromatographié sur silice (éluant: mélange dichlorométhane-méthanol-ammoniaque 95:4,5:0,5).

Rendement: 52%. P.F.: 145-148 °C (acétate d'éthyle)

| Analyse pour $C_{13}H_{14}N_2O_2$ en %: | | | | | | |
|---|---|---|---|---|---|---|
| calculé<br>trouvé | C | 67,83<br>67,68 | H | 6,09<br>6,11 | N | 12,17<br>12,10 |

Le composé ainsi obtenu se présente sous la forme racémique. Il a été résolu en ses deux énantiomères par la méthode suivante.

On dissout à chaud 7,57 g (0,0329 mole) de 4-[1-(4H-1,3-benzodioxin-8-yl)éthyl]-1H-imidazole racémique dans 263 ml d'alcool isopropylique. On y ajoute 2,47 g (0,0165 mole) d'acide d-tartrique dissous dans 33 ml d'alcool isopropylique. Il se forme un précipité floconneux. On agite la suspension pendant 16 heures à la température ambiante puis on élimine la phase liquide par décantation. On reprend le résidu obtenu dans 300 ml d'alcool isopropylique à l'ébullition. La fraction insoluble cristallise. On refroidit à 45 °C et on agite encore le mélange à cette température pendant 4 heures. Ensuite on filtre à chaud (45 °C). On obtient 3,21 g d'un sel (hémitartrate) cristallin.

P.F.: 164 °C. $[alpha]_D^{25}$ = + 40,8 (c = 1, méthanol).

Le filtrat provenant de la séparation des cristaux sera traité au point b) ci-dessous.

a) Les cristaux obtenus ci-dessus sont d'abord recristallisés deux fois dans de l'alcool isopropylique.
P.F.: 178 °C. $[alpha]_D^{25}$ = +53,3 (c = 1, méthanol).
Ensuite, on dissout 1 g de ce sel dans 33 ml d'eau et on alcalinise la solution à pH 9,5 par addition d'une solution aqueuse d'hydroxyde de sodium 1N. Il se forme un précipité qui est filtré et recristallisé dans 1 ml d'acétate d'éthyle. On obtient ainsi 0,457 g de l'isomère dextrogyre d-4-[1-(4H-1,3-benzodioxin-8-yl) éthyl]-1H-imidazole. P.F.: 113,7 °C. $[alpha]_D^{25}$ = +69,8 (c = 1, méthanol).

| Analyse pour $C_{13}H_{14}N_2O_2$ en %: | | | | | | |
|---|---|---|---|---|---|---|
| calculé<br>trouvé | C | 67,83<br>67,88 | H | 6,09<br>6,06 | N | 12,17<br>12,13 |

b) Le filtrat est évaporé sous pression réduite pour éliminer l'alcool isopropylique. On dissout le résidu obtenu dans de l'eau et on alcalinise la solution à pH 9,5 par addition d'une solution aqueuse d'hydroxyde de sodium 1N. La base liberée précipite, elle est filtrée puis traitée dans de l'alcool isopropylique par un demi-équivalent d'acide l-tartrique. Le sel formé précipite dans le milieu. On filtre et on recristallise ce sel dans de l'alcool isopropylique. P.F.: 178 °C. $[alpha]_D^{25}$ = -51,57 (c = 1, méthanol).
Ensuite, on le redissout dans de l'eau et on alcalinise la solution à pH 9,5 par additon d'une solution aqueuse d'hydroxyde de sodium 1N. Il se forme un précipité qui est filtré et recristallisé deux fois dans de l'acétate d'éthyle. On obtient 0,296 g de l'isomère lévogyre l-4-[1-(4H-1,3-benzodioxin-8-yl)-éthyl]-1H-imidazole.
P.F.: 114,4 °C. $[alpha]_D^{25}$ = -72,6 (c = 1, méthanol).

| Analyse pour $C_{13}H_{14}N_2O_2$ en %: | | | | | | |
|---|---|---|---|---|---|---|
| calculé<br>trouvé | C | 67,83<br>67,78 | H | 6,09<br>6,10 | N | 12,17<br>12,24 |

6. chlorhydrate de 4-[(6-méthyl-4H-1,3-benzodioxin-8-yl)méthyl]-1H-imidazole.

Ce composé a été préparé de manière analogue.

P.F.: 175-183 °C (décomposition).

| Analyse pour $C_{13}H_{14}N_2O_2$ . HCl en %: | | | | | | |
|---|---|---|---|---|---|---|
| calculé<br>trouvé | C | 58,58<br>56,47 | H | 5,63<br>5,63 | N | 10,51<br>9,93 |

Exemple 5. Préparation des 4-[1-[2,2-diméthyl-4H-1,3-benzodioxin-6 (ou 8)-yl]alkyl]-1H-imidazoles de formules I et III ($Z_1 + Z_2 = -C(CH_3)_2-$).

1. 4-[(2,2-diméthyl-4H-1,3-benzodioxin-6-yl)méthyl]-1H-imidazole.

On hydrogénolyse 3,4 g (0,013 mole) d'alpha-(2,2-diméthyl-4H-1,3-benzodioxin-6-yl)-1H-imidazole-4-méthanol (préparé à l'exemple 3.4.) dans du méthanol à 80 °C pendant 5 heures, en présence de charbon palladié à 10% et sous une pression d'hydrogène de 2,8 bars. Puis on filtre le catalyseur et on élimine le solvant sous pression réduite. Le résidu obtenu est chromatographié sur silice (éluant: dichlorométhane-méthanol 90:10). On obtient 3 g de 4-[(2,2-diméthyl-4H-1,3-benzodioxin-6-yl)méthyl]-1H-imidazole.

Rendement: 94%. P.F.: 150-170 °C.

Spectre RMN (DMSO): delta 1,45 (6H, s, $CH_3$-C-$CH_3$), 3,78 (2H, s, $CH_2$), 4,77 (2H, s, $CH_2$), 6,5-7,7 (5H, m, ArH + ImH).

2.a. 4-[(2,2-diméthyl-4H-1,3-benzodioxin-8-yl)méthyl]-1H-imidazole.

On introduit 94,13 g (0,317 mole) de chlorhydrate d'alpha-(2,2-diméthyl-4H-1,3-benzodioxin-8-yl)-1H-imidazole-4-méthanol (préparé à l'exemple 3.1. ou 3.5.) dans un litre d'ammoniac liquide anhydre. On complète la solubilisation du réactif par addition d'un litre de tétrahydrofuranne. On ajoute ensuite par morceaux 14,6 g de sodium (0,634 mole). Cinq minutes après la disparition du sodium, on ajoute 34 g de chlorure d'ammonium et on maintient l'agitation du milieu réactionnel pendant une demi-heure. On ajoute 1,56 litre d'une solution à 10% de méthanol dans du toluène. On chasse ensuite l'ammoniac au bain-marie. On ajoute encore 780 ml d'eau et l'on sépare la phase organique. La phase aqueuse est extraite deux fois par 500 ml de toluène. Les phases organiques sont rassemblées et lavées par 1 litre d'eau, puis séchées sur du sulfate de sodium et distillées sous pression réduite.

On recristallise le résidu obtenu dans 900 ml d'acétone à l'ébullition. On obtient ainsi 57,8 g de 4-[(2,2-diméthyl-4H-1,3-benzodioxin-8-yl)méthyl]-1H-imidazole fondant entre 160 et 170 °C.

Rendement: 74,6%.

| Analyse pour $C_{14}H_{16}N_2O_2$ en %: | | | | | | |
|---|---|---|---|---|---|---|
| calculé<br>trouvé | C | 68,85<br>69,15 | H | 6,56<br>6,70 | N | 11,48<br>11,53 |

2.b. Le même produit peut être préparé au départ de 0,8 g (0,0015 mole) d'alpha-(6-chloro-2,2-diméthyl-4H-1,3-benzodioxin-8-yl)-1-triphénylméthyl-1H-imidazole-4-méthanol (obtenu à l'exemple 1.C.3.) et 0,245 g (0,0106 mole) de sodium selon le procédé décrit à l'exemple 3.5. Le résidu obtenu après évaporation de la phase toluénique est chromatographié sur 150 g de silice (10 mym) (éluant: acétate d'éthyle-méthanol 95:5). On obtient 123 mg de produit conforme à celui isolé au point 2.a. ci-dessus.

Les produits suivants ont été préparés par la méthode décrite au point 2.a. ci-dessus:

3. 4-[(2,2-diméthyl-4H-1,3-benzodioxin-8-yl)méthyl]-1-méthyl-1H-imidazole.

Ce composé a été préparé au départ du chlorhydrate d'alpha-(2,2-diméthyl-4H-1,3-benzodioxin-8-yl)-1-méthyl-1H-imidazole-4-méthanol (préparé à l'exemple 1.C.12.). Le résidu obtenu est purifié par

chromatographie sur silice (éluant: dichlorométhane-méthanol: 98:2). Rendement: 67%. P.F.: 67-72 ° C (éther isopropylique-hexane).

| Analyse pour $C_{15}H_{18}N_2O_2$ en %: | | | | | | |
|---|---|---|---|---|---|---|
| calculé<br>trouvé | C | 69,74<br>69,93 | H | 7,02<br>7,54 | N | 10,84<br>10,78 |

4. 4-[(2,2-diméthyl-4H-1,3-benzodioxin-8-yl)méthyl]-5-méthyl-1H-imidazole.

Ce composé a été préparé à partir de 9,68 g du mélange des chlorhydrates des dérivés O-méthylé et alcoolique préparé à l'exemple 3.2. Il a été purifié par chromatographie sur silice (éluant: dichlorométhane-méthanol 90:10). On obtient 4 g du produit désiré.
P.F.: 172-178 ° C (dichlorométhane).

| Analyse pour $C_{15}H_{18}N_2O_2$ en %: | | | | | | |
|---|---|---|---|---|---|---|
| calculé<br>trouvé | C | 69,74<br>67,55 | H | 7,02<br>6,98 | N | 10,84<br>10,38 |

5. 4-[(2,2,6-triméthyl-4H-1,3-benzodioxin-8-yl)méthyl]-1H-imidazole.

Ce composé a été préparé à partir de 2,3 g du mélange renfermant le dérivé O-méthylé préparé à l'exemple 3.3. On isole 0,9 g du produit désiré.
P.F.: 152-155 ° C (acétate d'éthyle).

| Analyse pour $C_{15}H_{18}N_2O_2$ en %: | | | | | | |
|---|---|---|---|---|---|---|
| calculé<br>trouvé | C | 69,74<br>69,82 | H | 7,02<br>7,04 | N | 10,84<br>10,64 |

6. 4-[1-(2,2-diméthyl-4H-1,3-benzodioxin-8-yl)éthyl]-1H-imidazole.

Ce composé a été préparé à partir du chlorhydrate d'alpha-(2,2-diméthyl-4H-1,3-benzodioxin-8-yl)-alpha-méthyl-1H-imidazole-4-méthanol (préparé à l'exemple 3.9.). Le résidu obtenu après évaporation du toluène cristallise par agitation à 60 ° C dans de l'éther isopropylique.
Rendement: 60%. P.F.: 118 et 130 ° C.

| Analyse pour $C_{15}H_{18}N_2O_2$ en %: | | | | | | |
|---|---|---|---|---|---|---|
| calculé<br>trouvé | C | 69,74<br>69,64 | H | 7,02<br>6,95 | N | 10,84<br>10,72 |

7. 4-[1-(2,2-diméthyl-4H-1,3-benzodioxin-8-yl)pentyl]-1H-imidazole.

Ce composé a été préparé par réduction d'alpha-n-butyl-alpha-(2,2-diméthyl-4H-1,3-benzodioxin-8-yl)-1H-imidazole-4-méthanol (préparé à l'exemple 3.8.).
Rendement: 23,6%. P.F.: 108-112 ° C (cyclohexane).

| Analyse pour $C_{18}H_{24}N_2O_2$ en %: | | | | | | |
|---|---|---|---|---|---|---|
| calculé<br>trouvé | C | 72,0<br>72,08 | H | 8,0<br>8,15 | N | 9,33<br>9,32 |

Exemple 6. Préparation des 3-[1-(1H-imidazol-4-yl)alkyl]-2 (ou 6)-hydroxybenzèneméthanols de formule I ($Z_1$ = $Z_2$ = H).

1. 3-[(1H-imidazol-4-yl)méthyl]-2-hydroxybenzèneméthanol.

82,83 g (0,339 mole) de 4-[(2,2-diméthyl-4H-1,3-benzodioxin-8-yl)-méthyl]-1H-imidazole (préparé à l'exemple 5.2.) sont mis en suspension dans 1645 ml d'eau (pH = 7,9) et dissous par additon en 80 minutes de 349 ml d'une solution aqueuse d'acide chlorhydrique 1N. Lorsque cette addition est terminée, le pH de la solution est 2,5. On chauffe ensuite le milieu réactionnel pendant une heure dans un bain d'huile à 120 °C. On refroidit la solution, on la traite avec 5 g de norite et on la filtre sur hyflo-cel. On alcalinise ensuite cette solution par addition de 242,5 ml d'une solution aqueuse d'hydroxyde de sodium 1N (pH = 8,5). On filtre le précipité, on le lave à l'eau et on l'essore. Puis, ce précipité est dissous dans 3,3 litres d'acétate d'éthyle en présence de norite et de quelques grammes de sulfate de sodium. On filtre, on concentre jusqu'à un volume de 400 ml et on laisse cristalliser. On obtient 55,67 g de 3-[(1H-imidazol-4-yl)méthyl]-2-hydroxybenzèneméthanol.
Rendement: 80,4%. P.F.: 152-155 °C.

| Analyse pour $C_{11}H_{12}N_2O_2$ en %: | | | | | | |
|---|---|---|---|---|---|---|
| calculé<br>trouvé | C | 64,69<br>65,05 | H | 5,92<br>5,69 | N | 13,72<br>13,72 |

Les composés suivants ont été préparés par la méthode décrite ci-dessus.
2. 3-[(1-méthyl-1H-imidazol-4-yl)méthyl]-2-hydroxybenzèneméthanol.
Ce composé a été préparé à partir de 7 g de 4-[(2,2-diméthyl-4H-1,3-benzodioxin-8-yl)méthyl]-1-méthyl-1H-imidazole (préparé à l'exemple 5.3.).
On obtient 5,6 g de produit souhaité.
Rendement: 73%. P.F.: 139-144 °C (acétonitrile).

| Analyse pour $C_{12}H_{14}N_2O_2$ en %: | | | | | | |
|---|---|---|---|---|---|---|
| calculé<br>trouvé | C | 66,03<br>65,81 | H | 6,46<br>6,65 | N | 12,83<br>12,55 |

3. 3-[(5-méthyl-1H-imidazol-4-yl)méthyl]-2-hydroxybenzèneméthanol.
Ce composé a été préparé à partir de 4 g de 4-[(2,2-diméthyl-4H-1,3-benzodioxin-8-yl)méthyl]-5-méthyl-1H-imidazole (préparé à l'exemple 5.4.). On obtient 2,7 g du produit souhaité.
Rendement: 80%. P.F.: 126-130 °C (acétonitrile).

| Analyse pour $C_{12}H_{14}N_2O_2$ en %: | | | | | | |
|---|---|---|---|---|---|---|
| calculé<br>trouvé | C | 66,03<br>66,25 | H | 6,46<br>6,44 | N | 12,83<br>12,76 |

4. 3-[(1H-imidazol-4-yl)méthyl]-2-hydroxy-5-méthylbenzèneméthanol.
Ce composé a été préparé à partir de 3 g de 4-[(2,2,6-triméthyl-4H-1,3-benzodioxin-8-yl)méthyl]-1H-imidazole (préparé à l'exemple 5.5.).
On obtient 2,4 g du produit souhaité.
Rendement: 95%. P.F.: 170-175 °C (décomposition).

| Analyse pour $C_{12}H_{14}N_2O_2$ en %: | | | | | | |
|---|---|---|---|---|---|---|
| calculé<br>trouvé | C | 66,03<br>66,02 | H | 6,46<br>6,56 | N | 12,83<br>12,73 |

5. 3-[1-(1H-imidazol-4-yl)éthyl]2-hydroxybenzèneméthanol.
Ce composé a été préparé par hydrolyse du 4-[1-(2,2-diméthyl-4H-1,3-benzodioxin-8-yl)éthyl]-1H-imidazole (préparé à l'exemple 5.6.).
Rendement: 60%. P.F.: 135-136 °C.

| Analyse pour $C_{12}H_{14}N_2O_2$ en %: | | | | | | |
|---|---|---|---|---|---|---|
| calculé<br>trouvé | C | 66,03<br>65,97 | H | 6,46<br>6,44 | N | 12,83<br>12,80 |

6. 3-[1-(1H-imidazol-4-yl)pentyl]-2-hydroxybenzèneméthanol.

Ce composé a été préparé par hydrolyse du 4-[1-(2,2-diméthyl-4H-1,3-benzodioxin-8-yl)pentyl]-1H-imidazole (préparé à l'exemple 5.7.). Rendement: 94%. P.F.: 93 °C.

| Analyse pour $C_{15}H_{20}N_2O_2$ en %: | | | | | | |
|---|---|---|---|---|---|---|
| calculé<br>trouvé | C | 69,23<br>69,30 | H | 7,69<br>7,70 | N | 10,77<br>10,81 |

7. 3-[(1H-imidazol-4-yl)méthyl]-6-hydroxybenzèneméthanol.

Ce composé a été préparé à partir de 4 g de 4-[(2,2-diméthyl-4H-1,3-benzodioxin-6-yl)méthyl]-1H-imidazole (préparé à l'exemple 5.1.). On obtient 1,6 g du produit souhaité.
Rendement: 48%. P.F.: 149-155 °C (décomposition).

| Analyse pour $C_{11}H_{12}N_2O_2$ en %: | | | | | | |
|---|---|---|---|---|---|---|
| calculé<br>trouvé | C | 64,70<br>65,11 | H | 5,88<br>6,11 | N | 13,72<br>13,45 |

Exemple 7. Préparation des 3-[(1H-imidazol-4-yl)méthyl]-2-hydroxybenzoates d'alkyle de départ de formule IV.

3-[(1H-imidazol-4-yl)méthyl]-2-hydroxybenzoate d'éthyle.

a. 3-[(1H-imidazol-4-yl)méthyl]-2-oxo-cyclohexanecarboxylate d'éthyle.

Sous atmosphère d'argon, on prépare une solution d'éthylate de sodium en dissolvant 58,2 g (2,53 moles) de sodium dans 2 litres d'éthanol absolu. On refroidit à 10 °C et on ajoute rapidement, sous vive agitation, 195,5 g (1,15 mole) de 2-oxo-cyclohexanecarboxylate d'éthyle. Le sel sodique précipite et le mélange est agité à 20 °C pendant une heure puis est refroidi vers -10 °C. On y ajoute goutte à goutte 193,5 g (1,26 mole) de chlorhydrate de 4-chlorométhyl-1H-imidazole en solution dans 1 litre d'éthanol absolu. La température du mélange réactionnel est maintenue à -10 °C pendant une heure puis on laisse revenir à température ambiante et on maintient l'agitation pendant 20 heures. On distille 2 litres de solvant à la pression atmosphérique et on ajoute, entre 30 et 40 °C, une solution d'éthylate de sodium formée par dissolution de 79,35 g (3,45 moles) de sodium dans 1,2 litre d'éthanol absolu. On poursuit la distillation du solvant jusqu'à ce que le milieu réactionnel atteigne une température de 100-105 °C. On arrête la distillation du solvant et on maintient le reflux pendant 10 heures. On refroidit à la température ambiante et on ajoute 200 ml d'éthanol absolu, puis on refroidit le mélange à -10 °C et on y introduit, goutte à goutte, 400 ml d'une solution méthanolique d'acide chlorhydrique 9,9 N. On laisse la température remonter à 10 °C. On élimine le chlorure de sodium par centrifugation. Après évaporation du solvant sous pression réduite, on obtient 180 g d'une huile brune. Le culot de chlorure de sodium séparé par centrifugation est lavé deux fois au moyen d'un litre de dichlorométhane. Ce solvant est ensuite éliminé sous pression réduite et on récupère encore 60 g d'huile.

L'huile est purifiée par chromatographie sur silice (colonne 80 mm diamètre, silice Merck TLC 60 H 15 mym; éluant: mélange dichlorométhane-éthanol-ammoniaque 93:6,5:0,5). On obtient finalement 138 g de 3-[(1H-imidazol-4-yl)méthyl]-2-oxo-cyclohexanecarboxylated'éthyle sous forme d'une huile qui solidifie lentement (mélange de diastéréoisomères).
Rendement: 48%.

Le 2-oxo-cyclohexanecarboxylate d'éthyle utilisé comme produit de départ a été préparé selon la méthode décrite par H.R. SNYDER et al. (Organic Syntheses, Coll. Vol.II,(1943),531-534).

Le chlorhydrate de 4-chlorométhyl-1H-imidazole utilisé comme produit de départ a été préparé selon la méthode décrite par R.A. TURNER et al. (J.Am.chem.Soc.71,(1949),2801-2803).

b. 3-[(1H-imidazol-4-yl)méthyl]-2-hydroxybenzoate d'éthyle.

A une solution contenant 37,5 g (0,55 mole) de 3-[(1H-imidazol-4-yl)méthyl]-2-oxo-cyclohexanecarboxylate d'éthyle dans 1,5 litre d'acide acétique glacial, on ajoute goutte à goutte en 30 minutes, entre 17 et 20 °C et sous atmosphère d'argon, 176 g (56,5 ml, 1,1 mole) de brome en solution dans 400 ml d'acide acétique glacial. On chauffe ensuite le mélange à reflux pendant 4 heures. La solution rouge noircit progressivement. On refroidit le mélange à 20 °C et on évapore sous pression réduite. Le résidu est repris dans 1,2 litre de solution aqueuse d'acide acétique à 50%. On y ajoute encore une pointe de spatule de résine échangeuse d'anions (Amberlite IR 45 acétate), 25 g de palladium sur carbone à 10% de palladium et 25 g de rhodium sur carbone à 10% de rhodium et on agite pendant 150 minutes sous une pression d'hydrogène de 3,5 bars. On filtre le mélange sur Célite et on l'évapore sous pression réduite. L'huile obtenue est dissoute dans 1 litre d'un mélange glace-eau, la solution est amenée à pH 8 par addition d'une solution aqueuse d'hydroxyde de sodium 2N et est rapidement extraite 5 fois par 400 ml d'acétate d'éthyle. Les phases organiques sont réunies, séchées sur du sulfate de magnésium, filtrées et évaporées sous pression réduite. Le résidu est purifié par chromatographie sur silice (éluant: mélange dichlorométhane-éthanol-ammoniaque 93,5:6:0,5). On obtient 75,9 g de 3-[(1H-imidazol-4-yl)-méthyl]-2-hydroxybenzoate d'éthyle sous forme d'une huile qui cristallise lentement.
Rendement: 55%. P.F.: 133 °C.

| Analyse pour $C_{13}H_{14}N_2O_3$ en %: | | | | | | |
|---|---|---|---|---|---|---|
| calculé<br>trouvé | C | 63,41<br>63,72 | H | 5,69<br>5,80 | N | 11,38<br>11,32 |

Exemple 8. Préparation des 3-[(1H-imidazol-4-yl)méthyl]-2-hydroxybenzèneméthanols de formule I ($Y_1$ = $OZ_2$, $Y_2$ = $Z_1$ = $Z_2$ = $R_5$ = H).

3-[1H-imidazol-4-yl)méthyl]-2-hydroxybenzèneméthanol.

A une solution de 73,9 g (0,3 mole) de 3-[(1H-imidazol-4-yl)méthyl]-2-hydroxybenzoate d'éthyle (préparé à l'exemple 7) dans 4 litres de tétrahydrofuranne anhydre, on ajoute par petites portions, sous atmosphère d'argon, 22,8 g (0,6 mole) d'hydrure de lithium-aluminium. La température s'élève progressivement et lorsque l'addition est terminée, on porte le mélange à reflux pendant 3 heures. On refroidit à 10 °C, et on ajoute goutte à goutte 200 ml d'acétate d'éthyle. On évapore sous pression réduite et on reprend le résidu dans 1 litre d'un mélange glace-eau. La suspension est amenée à pH 7,5 par addition d'acide chlorhydrique concentré. On ajoute 700 ml d'acétate d'éthyle et on agite vigoureusement pendant 30 minutes, puis on filtre. Le filtrat est décanté et la phase aqueuse est extraite 3 fois avec 500 ml d'acétate d'éthyle. On réunit les phases organiques, on les sèche sur du sulfate de magnésium, on les filtre, puis on les évapore sous pression réduite. On obtient 61 g d'une huile qui cristallise. Le gâteau de filtration est soigneusement lavé avec de l'acétate d'éthyle.

On reprend ce gâteau par 1,5 litre d'acétate d'éthyle et on l'extrait à reflux pendant 3 heures. On filtre et après évaporation du solvant sous pression réduite, on recueille encore 7 g d'huile. Le produit est purifié par chromatographie sur silice (éluant: mélange dichlorométhane-éthanol-ammoniaque 91,5:8:0,5) et recristallisation dans l'acétate d'éthyle. On obtient 33,8 g de 3-[(1H-imidazol-4-yl)méthyl]-2-hydroxybenzèneméthanol, identique au produit obtenu à l'exemple 6.1.
Rendement: 55%.

**Revendications**
**Revendications pour les Etats contractants suivants : AT, BE, CH, DE, FR, GB, IT, LI, LU, NL, SE**

**1.** 1H-Imidazoles substitués, leurs isomères optiques et leurs mélanges racémiques répondant à la formule générale:

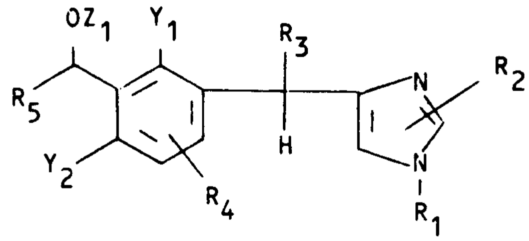

(I)

dans laquelle

| | |
|---|---|
| $R_1$, $R_2$, $R_3$ et $R_5$, | qui peuvent être identiques ou différents, représentent chacun un atome d'hydrogène ou un radical alkyle contenant 1 à 4 atomes de carbone, |
| $R_4$ | représente un atome d'hydrogène, un radical alkyle contenant 1 à 4 atomes de carbone ou un radical alkoxy contenant 1 à 4 atomes de carbone, |
| un des radicaux $Y_1$ et $Y_2$ | représente de l'hydrogène et l'autre un radical $OZ_2$, et |
| $Z_1$ et $Z_2$ | pris isolément représentent tous deux de l'hydrogène ou un radical alkyle contenant 1 à 4 atomes de carbone et ensemble un groupe $-CH_2-$ ou $-C(CH_3)_2-$, |

ainsi que leurs sels d'addition d'acides non toxiques pharmaceutiquement acceptables.

**2.** 1H-Imidazoles répondant à la formule générale I donnée dans la revendication 1, dans laquelle

| | |
|---|---|
| $R_1$, $R_2$, $R_4$ et $R_5$ | représentent de l'hydrogène, |
| $R_3$ | représente de l'hydrogène ou un radical alkyle contenant 1 à 4 atomes de carbone, et |
| $Z_1$ et $Z_2$ | pris isolément représentent de l'hydrogène et ensemble un groupe $-CH_2-$ ou $-C(CH_3)_2-$, |

et leurs sels d'addition d'acides non toxiques pharmaceutiquement acceptables.

**3.** 3-[(1H-imidazol-4-yl)méthyl]-2-hydroxybenzèneméthanol et ses sels d'addition d'acides non toxiques pharmaceutiquement acceptables selon la revendication 1.

**4.** 3-[(1H-imidazol-4-yl)méthyl]-6-hydroxybenzèneméthanol et ses sels d'addition d'acides non toxiques pharmaceutiquement acceptables selon la revendication 1.

**5.** 3-[1-(1H-imidazol-4-yl)éthyl]-2-hydroxybenzèneméthanol et ses sels d'addition d'acides non toxiques pharmaceutiquement acceptables selon la revendication 1.

**6.** 3-[1-(1H-imidazol-4-yl)pentyl]-2-hydroxybenzèneméthanol et ses sels d'addition d'acides non toxiques pharmaceutiquement acceptables selon la revendication 1.

**7.** 4-[(4H-1,3-benzodioxin-8-yl)méthyl]-1H-imidazole et ses sels d'addition d'acides non toxiques pharmaceutiquement acceptables selon la revendication 1.

**8.** 4-[1-(4H-1,3-benzodioxin-8-yl)éthyl]-1H-imidazole et ses sels d'addition d'acides non toxiques pharmaceutiquement acceptables selon la revendication 1.

**9.** 4-[(2,2-diméthyl-4H-1,3-benzodioxin-8-yl)méthyl]-1H-imidazole et ses sels d'addition d'acides non toxiques pharmaceutiquement acceptables selon la revendication 1.

**10.** 4-[1-(2,2-diméthyl-4H-1,3-benzodioxin-8-yl)éthyl]-1H-imidazole et ses sels d'addition d'acides non toxiques pharmaceutiquement acceptables selon la revendication 1.

**11.** 4-[1-(2,2-diméthyl-4H-1,3-benzodioxin-8-yl)pentyl]-1H-imidazole et ses sels d'addition d'acides non toxiques pharmaceutiquement acceptables selon la revendication 1.

**12.** Procédé de préparation de 1H-imidazoles substitués et de leurs sels tels que définis dans la revendication 1, caractérisé en ce que:

a) pour préparer les 1H-imidazoles répondant à la formule générale I dans laquelle $Z_1$ et $Z_2$ pris isolément représentent un radical alkyle contenant 1 à 4 atomes de carbone et pris ensemble représentent un group $-CH_2-$ ou $-C(CH_3)_2-$, on réduit un composé imidazolique de formule

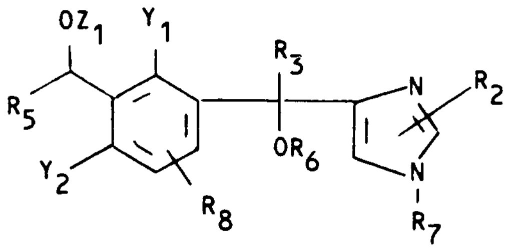

(II)

dans laquelle $R_2$, $R_3$ et $R_5$ ont la signification donnée à la revendication 1, $R_6$ représente un atome d'hydrogène ou un radical alkyle ayant 1 à 4 atomes de carbone, $R_7$ représente un radical $R_1$ tel que défini dans la revendication 1 ou un radical aisément séparable par réduction, $R_8$ représente un radical $R_4$ tel que défini dans la revendication 1 ou un atome de chlore, un des radicaux $Y_1$ et $Y_2$ représente de l'hydrogène et l'autre un radical $OZ_2$, $Z_1$ et $Z_2$ ayant la signification donnée ci-dessus; ou

b) pour préparer les 1H-imidazoles répondant à la formule générale I dans laquelle $Z_1$ et $Z_2$ sont de l'hydrogène, on hydrolyse en milieu acide aqueux un 4-[[2,2-diméthyl-4H-1,3-benzodioxin-6(ou 8)-yl]méthyl]-1H-imidazole de formule

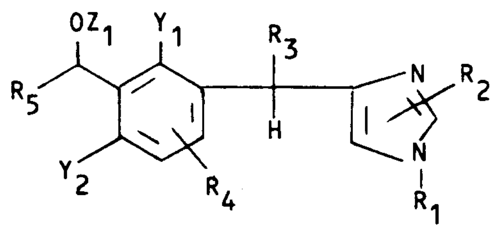

(III)

dans laquelle $R_1$, $R_2$, $R_3$, $R_4$ et $R_5$ ont la signification donnée à la revendication 1, un des radicaux $Y_1$ et $Y_2$ représente de l'hydrogène et l'autre un radical $OZ_2$, $Z_1$ et $Z_2$ étant ensemble le groupe $-C-(CH_3)_2-$; ou

c) pour préparer les 1H-imidazoles répondant à la formule générale I dans laquelle $Y_1$ = $OZ_2$ et $Y_2$, $Z_1$, $Z_2$ et $R_5$ sont de l'hydrogène, on réduit un 3-[(1H-imidazol-4-yl)méthyl]-2-hydroxybenzoate d'alkyle de formule

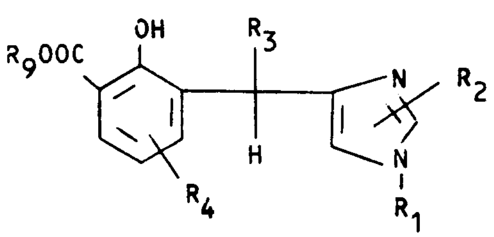

(IV)

dans laquelle $R_1$, $R_2$, $R_3$ et $R_4$ ont la signification donnée à la revendication 1 et $R_9$ représente un radical alkyle contenant 1 à 4 atomes de carbone; et

d) en ce que éventuellement, on convertit les 1H-imidazoles de formule I ainsi obtenus en leurs sels d'addition d'acides non toxiques pharmaceutiquement acceptables.

**13.** 1H-imidazoles selon l'une quelconque des revendications 1 à 11 ou un de leurs sels d'addition d'acides non toxiques pharmaceutiquement acceptables pour utilisation en thérapie comme agent anti-ischémique.

**14.** Compositions thérapeutiques renfermant une quantité thérapeutiquement efficace d'un 1H-imidazole selon l'une quelconque des revendications 1 à 11 ou d'un de ses sels d'addition d'acides non toxiques pharmaceutiquement acceptables, en association avec des excipients pharmaceutiques solides ou liquides.

**Revendication pour les Etats contractants suivants: ES, GR**

**1.** Procédé de préparation de 1H-imidazoles substitués, leurs isomères optiques et leurs mélanges racémiques répondant à la formule générale

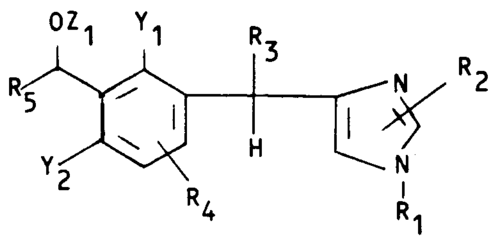

(I)

dans laquelle

| | |
|---|---|
| $R_1$, $R_2$, $R_3$ et $R_5$, | qui peuvent être identiques ou différents, représentent chacun un atome d'hydrogène ou un radical alkyle contenant 1 à 4 atomes de carbone, |
| $R_4$ | représente un atome d'hydrogène, un radical alkyle contenant 1 à 4 atomes de carbone ou un radical alkoxy contenant 1 à 4 atomes de carbone, |
| un des radicaux $Y_1$ et $Y_2$ | représente de l'hydrogène et l'autre un radical $OZ_2$, et |
| $Z_1$ et $Z_2$ | pris isolément représentent tous deux de l'hydrogène ou un radical alkyle contenant 1 à 4 atomes de carbone et ensemble un groupe $-CH_2-$ ou $-C(CH_3)_2-$, |

ainsi que leurs sels d'addition d'acides non toxiques pharmaceutiquement acceptables, caractérisé en ce que:

a) pour préparer les 1H-imidazoles répondant à la formule générale I dans laquelle $Z_1$ et $Z_2$ pris isolément représentent un radical alkyle contenant 1 à 4 atomes de carbone et pris ensemble représentent un groupe $-CH_2-$ ou $-C(CH_3)_2-$, on réduit un composé imidazolique de formule

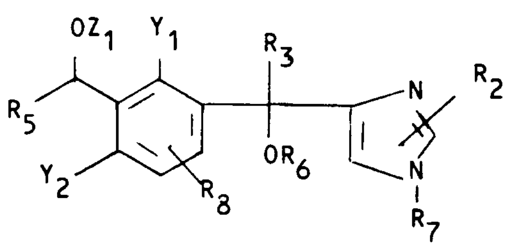

(II)

dans laquelle $R_2$, $R_3$ et $R_5$ ont la signification donnée ci-dessus, $R_6$ représente un atome d'hydrogène ou un radical alkyle ayant 1 à 4 atomes de carbone, $R_7$ représente un radical $R_1$ tel que défini ci-dessus ou un radical aisément séparable par réduction, $R_8$ représente un radical $R_4$ tel que défini ci-dessus ou un atome de chlore, un des radicaux $Y_1$ et $Y_2$ représente de l'hydrogène et l'autre un radical $OZ_2$, $Z_1$ et $Z_2$ ayant le signification donnée ci-dessus; ou

b) pour préparer les 1H-imidazoles répondant à la formule générale I dans laquelle $Z_1$ et $Z_2$ sont de l'hydrogène, on hydrolyse en milieu acide aqueux un 4-[[2,2-diméthyl-4H-1,3-benzodioxin-6(ou 8)yl]-

méthyl]-1H-imidazole de formule

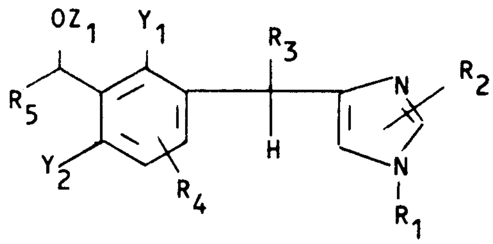

(III)

dans laquelle $R_1$, $R_2$, $R_3$, $R_4$ et $R_5$ ont la signification donnée ci-dessus, un des radicaux $Y_1$ et $Y_2$ représente de l'hydrogène et l'autre un radical $OZ_2$, $Z_1$ et $Z_2$ étant ensemble le groupe $-C(CH_3)_2-$; ou

c) pour préparer les 1H-imidazoles répondant à la formule générale I dans laquelle $Y_1$ = $OZ_2$ et $Y_2$, $Z_1$, $Z_2$ et $R_5$ sont de l'hydrogène, on réduit un 3-[(1H-imidazol-4-yl)méthyl]-2-hydroxybenzoate d'alkyle de formule

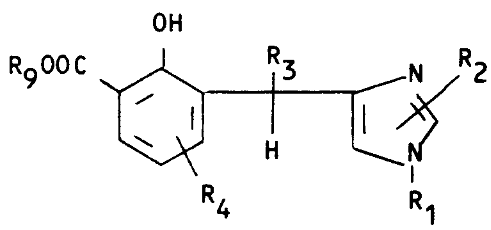

(IV)

dans laquelle $R_1$, $R_2$, $R_3$ et $R_4$ ont la signification donnée ci-dessus et $R_9$ représente un radical alkyle contenant 1 à 4 atomes de carbone; et

d) en ce que éventuellement, on convertit les 1H-imidazoles de formule I ainsi obtenus en leurs sels d'addition d'acides non toxiques pharmaceutiquement acceptables.

**Claims**

**Claims for the following Contracting States: AT, BE, CH, DE, FR, GB, IT, LI, LU, NL, SE**

**1.** Substituted 1H-imidazoles, their optical isomers and their racemic mixtures, having the general formula

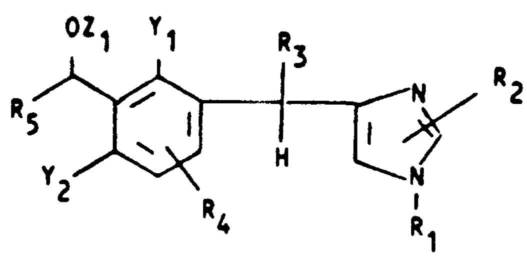

(I)

wherein

| | |
|---|---|
| $R_1$, $R_2$, $R_3$ and $R_5$, | which may be the same or different, each represent a hydrogen atom or an alkyl radical having 1 to 4 carbon atoms, |
| $R_4$ | represents a hydrogen atom, an alkyl radical having 1 to 4 carbon atoms or an alkoxy radical having 1 to 4 carbon atoms, |
| one of the radicals $Y_1$ and $Y_2$ | represents hydrogen and the other an $OZ_2$ radical, and |
| $Z_1$ and $Z_2$ | taken separately represent both hydrogen or an alkyl radical having 1 to 4 carbon atoms and taken together represent a $-CH_2-$ or $-C(CH_3)_2-$ group, |

as well as their non-toxic pharmaceutically acceptable acid addition salts.

**2.** 1H-imidazoles having the general formula I given in claim 1, wherein

| | |
|---|---|
| $R_1$, $R_2$, $R_4$ and $R_5$ | represent a hydrogen atom, |
| $R_3$ | represents a hydrogen atom or an alkyl radical having 1 to 4 carbon atoms, and |
| $Z_1$ and $Z_2$ | taken separately each represent a hydrogen atom and taken together represent a $-CH_2-$ or $-C(CH_3)_2-$ group |

as well as their non-toxic pharmaceutically acceptable acid addition salts.

**3.** 3-[(1H-imidazol-4-yl)methyl]-2-hydroxybenzenemethanol and its non-toxic pharmaceutically acceptable acid addition salts according to claim 1.

**4.** 3-[(1H-imidazol-4-yl)methyl]-6-hydroxybenzenemethanol and its non-toxic pharmaceutically acceptable acid addition salts according to claim 1.

**5.** 3-[1-(1H-imidazol-4-yl)ethyl]-2-hydroxybenzenemethanol and its non-toxic pharmaceutically acceptable acid addition salts according to claim 1.

**6.** 3-[1-(1H-imidazol-4-yl)pentyl]-2-hydroxybenzenemethanol and its non-toxic pharmaceutically acceptable acid addition salts according to claim 1.

**7.** 4-[(4H-1,3-benzodioxin-8-yl)methyl]-1H-imidazole and its non-toxic pharmaceutically acceptable acid addition salts according to claim 1.

**8.** 4-[1-(4H-1,3-benzodioxin-8-yl)ethyl]-1H-imidazole and its non-toxic pharmaceutically acceptable acid addition salts according to claim 1.

**9.** 4-[(2,2-dimethyl-4H-1,3-benzodioxin-8-yl)methyl]-1H-imidazole and its non-toxic pharmaceutically acceptable acid addition salts according to claim 1.

**10.** 4-[1-(2,2-dimethyl-4H-1,3-benzodioxin-8-yl)ethyl]-1H-imidazole and its non-toxic pharmaceutically acceptable acid addition salts according to claim 1.

**11.** 4-[1-(2,2-dimethyl-4H-1,3-benzodioxin-8-yl)pentyl]-1H-imidazole and its non-toxic pharmaceutically acceptable acid addition salts according to claim 1.

**12.** Process for the preparation of substituted 1H-imidazoles and their salts as defined in claim 1, characterised in that:

a) in order to prepare the 1H-imidazoles having the general formula I in which $Z_1$ and $Z_2$ taken separately represent an alkyl radical having 1 to 4 carbon atoms and taken together represent a $-CH_2-$ or $-C(CH_3)_2-$ group, an imidazole compound of the formula

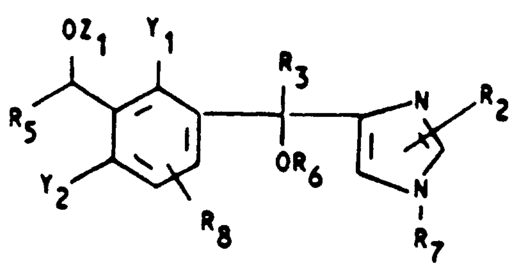

(II)

wherein $R_2$, $R_3$ and $R_5$ have the meanings given in claim 1, $R_6$ represents a hydrogen atom or an alkyl radical having 1 to 4 carbon atoms, $R_7$ represents an $R_1$ radical as defined in claim 1 or is a radical which can easily be removed by reduction, $R_8$ represents an $R_4$ radical as defined in claim 1 or is a chlorine atom, one of the radicals $Y_1$ and $Y_2$ represents hydrogen and the other an $OZ_2$

radical, $Z_1$ and $Z_2$ having the meanings given above, is reduced; or

b) in order to prepare the 1H-imidazoles having the general formula I in which $Z_1$ and $Z_2$ are hydrogen, a 4-[[2,2-dimethyl-4H-1,3-benzodioxin-6(or 8)-yl]methyl]-1H-imidazole of the formula

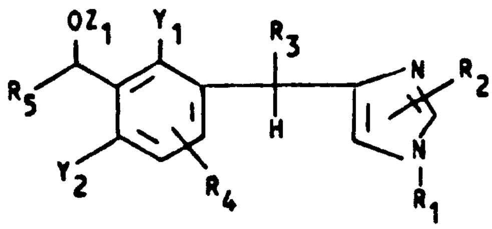

(III)

wherein $R_1$, $R_2$, $R_3$, $R_4$ and $R_5$ have the meanings given in claim 1, one of the radicals $Y_1$ and $Y_2$ represents hydrogen and the other an $OZ_2$ radical, $Z_1$ and $Z_2$ being together the $-C(CH_3)_2-$ group is hydrolysed in an aqueous acid medium; or

c) in order to prepare the 1H-imidazoles having the general formula I in which $Y_1 = OZ_2$ and $Y_2$, $Z_1$, $Z_2$ and $R_3$ are hydrogen, an alkyl 3-[(1H-imidazol-4-yl)methyl]-2-hydroxybenzoate of the formula

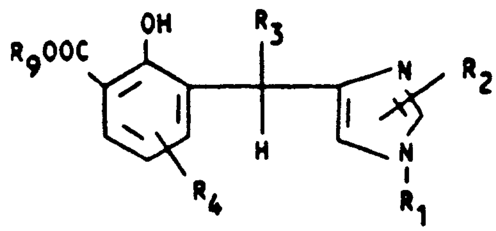

(IV)

wherein $R_1$, $R_2$, $R_3$ and $R_4$ have the meanings given in claim 1 and $R_9$ represents an alkyl radical having 1 to 4 carbon atoms, is reduced; and

d) optionally, the 1H-imidazoles of the formula I thus obtained are converted into their non-toxic pharmaceutically acceptable acid addition salts.

**13.** 1H-imidazoles according to any of claims 1 to 11 or one of their non-toxic pharmaceutically acceptable acid addition salts for use in therapy as anti-ischemic agent.

**14.** Therapeutic compositions containing a therapeutically effective amount of a 1H-imidazole according to any of claims 1 to 11 or one of its non-toxic pharmaceutically acceptable acid addition salts, in association with solid or liquid pharmaceutical excipients.

**Claim for the following Contracting States: ES, GR**

**1.** Process for the preparation of substituted 1H-imidazoles, their optical isomers and their racemic mixtures, having the general formula

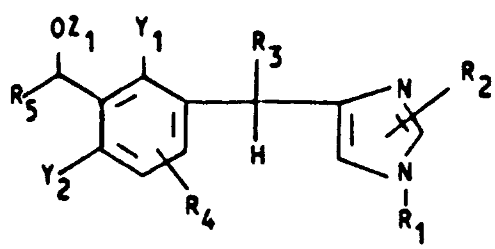

(I)

wherein

$R_1$, $R_2$, $R_3$ and $R_5$, which may be the same or different, each represent a hydrogen atom or an alkyl radical having 1 to 4 carbon atoms,

$R_4$ represents a hydrogen atom, an alkyl radical having 1 to 4 carbon atoms or an alkoxy radical having 1 to 4 carbon atoms,

one of the radicals $Y_1$ and $Y_2$ represents hydrogen and the other an $OZ_2$ radical, and

$Z_1$ and $Z_2$ taken separately represent both hydrogen or an alkyl radical having 1 to 4 carbon atoms and taken together represent a $-CH_2-$ or $-C(CH_3)_2-$ group,

as well as their non-toxic pharmaceutically acceptable acid addition salts, characterised in that:

a) in order to prepare the 1H-imidazoles having the general formula I in which $Z_1$ and $Z_2$ taken separately represent an alkyl radical having 1 to 4 carbon atoms and taken together represent a $-CH_2-$ or $-C(CH_3)_2-$ group, an imidazole compound of the formula

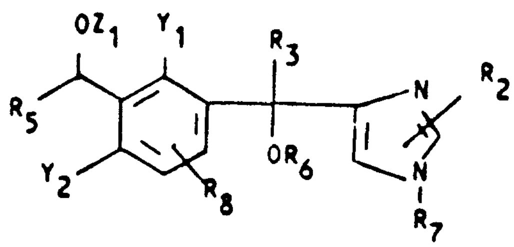

(II)

wherein $R_2$, $R_3$ and $R_5$ have the meanings given above, $R_6$ represents a hydrogen atom or an alkyl radical having 1 to 4 carbon atoms, $R_7$ represents an $R_1$ radical as defined above or is a radical which can easily be removed by reduction, $R_8$ represents an $R_4$ radical as defined above or is a chlorine atom, one of the radicals $Y_1$ and $Y_2$ represents hydrogen and the other an $OZ_2$ radical, $Z_1$ and $Z_2$ having the meanings given above, is reduced; or

b) in order to prepare the 1H-imidazoles having the general formula I in which $Z_1$ and $Z_2$ are hydrogen, a 4-[[2,2-dimethyl-4H-1,3-benzodioxin-6(or 8)-yl]methyl]-1H-imidazole of the formula

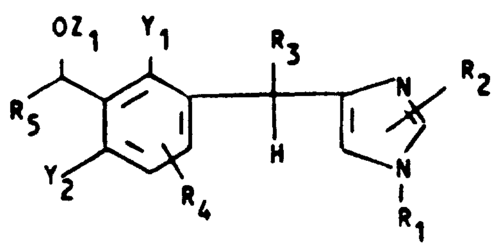

(III)

wherein $R_1$, $R_2$, $R_3$, $R_4$ and $R_5$ have the meanings given above, one of the radicals $Y_1$ and $Y_2$ represents hydrogen and the other an $OZ_2$ radical, $Z_1$ and $Z_2$ being together the $-C(CH_3)_2-$ group, is hydrolysed in an aqueous acid medium; or

c) in order to prepare the 1H-imidazoles having the general formula I in which $Y_1 = OZ_2$ and $Y_2$, $Z_1$ $Z_2$ and $R_3$ are hydrogen, an alkyl 3-[(1H-imidazol-4-yl)methyl]-2-hydroxybenzoate of the formula

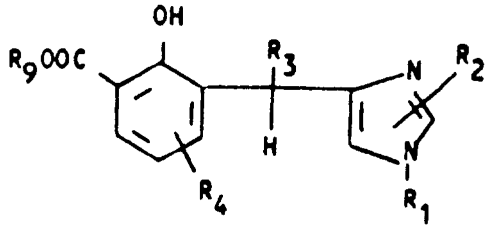

(IV)

wherein $R_1$, $R_2$, $R_3$ and $R_4$ have the meanings given above and $R_9$ represents an alkyl radical

having 1 to 4 carbon atoms, is reduced; and

d) optionally, the 1H-imidazoles of the formula I thus obtained are converted into their non-toxic pharmaceutically acceptable acid addition salts.

## Patentansprüche

**Patentansprüche für folgende Vertragsstaaten: AT, BE, CH, DE, FR, GB, IT, LI, LU, NL, SE**

1. Substituierte 1H-Imidazole, deren optische Isomeren und deren racemischen Mischungen, der allgemeinen Formel:

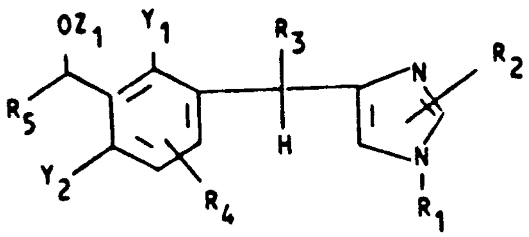

(I)

in der

| | |
|---|---|
| $R_1$, $R_2$, $R_3$ und $R_5$, | die gleich oder unterschiedlich sein können, jeweils ein Wasserstoffatom oder einen Alkylrest mit 1 bis 4 Kohlenstoffatomen darstellen, |
| $R_4$ | ein Wasserstoffatom, einen Alkylrest mit 1 bis 4 Kohlenstoffatomen oder einen Alkoxyrest mit 1 bis 4 Kohlenstoffatomen darstellt, |
| einer der Reste $Y_1$ und $Y_2$ | Wasserstoff und der andere einen $OZ_2$-Rest darstellt, und |
| $Z_1$ und $Z_2$ | getrennt genommen, alle beide Wasserstoff oder einen Alkylrest mit 1 bis 4 Kohlenstoffatomen und zusammen eine $-CH_2-$ oder $-C(CH_3)_2-$ Gruppe darstellen, |

sowie deren Additions-Salze von nicht toxischen, pharmazeutisch annehmbaren Säuren.

2. 1H-Imidazole, die der in Anspruch 1 gegebenen allgemeinen Formel I entsprechen, in der

| | |
|---|---|
| $R_1$, $R_2$, $R_4$ und $R_5$ | Wasserstoff darstellen, |
| $R_3$ | Wasserstoff oder einen Alkylrest mit 1 bis 4 Kohlenstoffatomen darstellt, und |
| $Z_1$ und $Z_2$, | getrennt genommen, Wasserstoff und zusammen eine $-CH_2-$ oder $-C(CH_3)_2-$ Gruppe darstellen, |

und deren Additions-Salze von nicht toxischen, pharmazeutisch annehmbaren Säuren.

3. 3-[(1H-Imidazol-4-yl)methyl]-2-hydroxybenzolmethanol und dessen Additions-Salze von nicht toxischen, pharmazeutisch annehmbaren Säuren gemäß Anspruch 1.

4. 3-[(1H-Imidazol-4-yl)methyl]-6-hydroxybenzolmethanol und dessen Additions-Salze von nicht toxischen, pharmazeutisch annehmbaren Säuren gemäß Anspruch 1.

5. 3-[1-(1H-Imidazol-4-yl)ethyl]-2-hydroxybenzolmethanol und dessen Additions-Salze von nicht toxischen, pharmazeutisch annehmbaren Säuren gemäß Anspruch 1.

6. 3-[1-(1H-Imidazol-4-yl)pentyl]-2-hydroxybenzolmethanol und dessen Additions-Salze von nicht toxischen, pharmazeutisch annehmbaren Säuren gemäß Anspruch 1.

7. 4-[(4H-1,3-Benzodioxin-8-yl)methyl]-1H-imidazol und dessen Additions-Salze von nicht toxischen, pharmazeutisch annehmbaren Säuren gemäß Anspruch 1.

8. 4-[1-(4H-1,3-Benzodioxin-8-yl)ethyl]-1H-imidazol und dessen Additions-Salze von nicht toxischen, pharmazeutisch annehmbaren Säuren gemäß Anspruch 1.

9. 4-[(2,2-Dimethyl-4H-1,3-benzodioxin-8-yl)methyl]-1H-imidazol und dessen Additions-Salze von nicht toxischen, pharmazeutisch annehmbaren Säuren gemäß Anspruch 1.

**10.** 4-[1-(2,2-Dimethyl-4H-1,3-benzodioxin-8-yl)ethyl]-1H-imidazol und dessen Additions-Salze von nicht toxischen, pharmazeutisch annehmbaren Säuren gemäß Anspruch 1.

**11.** 4-[1-(2,2-Dimethyl-4H-1,3-benzodioxin-8-yl)pentyl]-1H-imidazol und dessen Additions-Salze von nicht toxischen, pharmazeutisch annehmbaren Säuren gemäß Anspruch 1.

**12.** Verfahren zur Herstellung von substituierten 1H-Imidazolen und deren Salzen wie in Anspruch 1 definiert, dadurch gekennzeichnet, daß man

a) zur Herstellung der 1H-Imidazole der allgemeinen Formel I in der $Z_1$ und $Z_2$, getrennt genommen, einen Alkylrest mit 1 bis 4 Kohlenstoffatomen darstellen und, zusammen genommen, eine $-CH_2-$ oder $-C(CH_3)_2-$ Gruppe darstellen, eine Imidazolverbindung der Formel

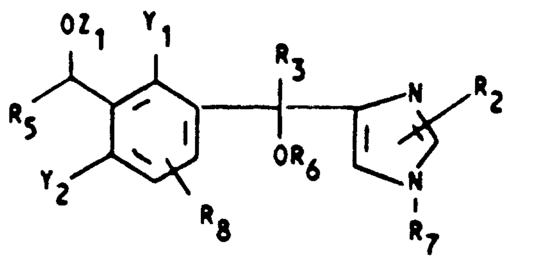

(II)

reduziert, in der $R_2$, $R_3$ und $R_5$ die in Anspruch 1 gegebene Bedeutung aufweisen, $R_6$ ein Wasserstoffatom oder einen Alkylrest mit 1 bis 4 Kohlenstoffatomen darstellt, $R_7$ einen $R_1$-Rest wie in Anspruch 1 definiert oder einen leicht durch Reduktion abtrennbaren Rest darstellt, $R_8$ einen $R_4$-Rest wie in Anspruch 1 definiert oder ein Chloratom darstellt, einer der Reste $Y_1$ und $Y_2$ Wasserstoff und der andere einen $OZ_2$-Rest darstellt, wobei $Z_1$ und $Z_2$ die oben genannte Bedeutung haben; oder

b) zur Herstellung der 1H-Imidazole der allgemeinen Formel I in der $Z_1$ und $Z_2$ Wasserstoff sind, in wässrigem, saurem Medium ein 4-[[2,2-Dimethyl-4H-1,3-benzodioxin-6(oder 8)-yl]methyl]-1H-imidazol der Formel

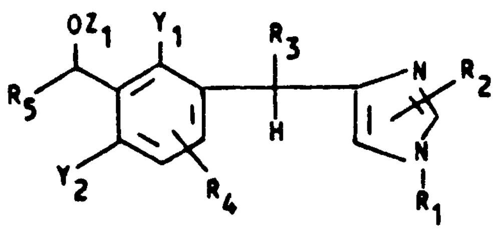

(III)

hydrolysiert, in der $R_1$, $R_2$, $R_3$, $R_4$ und $R_5$ die in Anspruch 1 gegebene Bedeutung aufweisen, einer der Reste $Y_1$ und $Y_2$ Wasserstoff und der andere einen $OZ_2$-Rest darstellt, wobei $Z_1$ und $Z_2$ zuzammen die $-C(CH_3)_2$-Gruppe sind; oder

c) zur Herstellung der 1H-Imidazole der allgemeinen Formel I in der $Y_1 = OZ_2$ und $Y_2$, $Z_1$, $Z_2$ und $R_5$ Wasserstoff sind, ein 3-[(1H-Imidazol-4-yl)methyl]-2-hydroxybenzoësäurealkylester der Formel

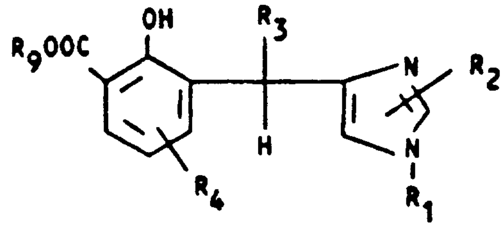

(IV)

reduziert, in der $R_1$, $R_2$, $R_3$ und $R_4$ die in Anspruch 1 gegebene Bedeutung aufweisen und $R_9$ einen Alkylrest mit 1 bis 4 Kohlenstoffatomen darstellt; und

d) gegebenfalls, die so erhaltenen 1H-Imidazole der Formel I in ihre Additions-Salze von nicht toxischen, pharmazeutisch annehmbaren Säuren umwandelt.

**13.** 1H-Imidazole nach einem der Ansprüche 1 bis 11 oder eines ihrer Additions-Salze von nicht toxischen, pharmazeutisch annehmbaren Säuren zur Verwendung bei der Therapie als anti-ischämisches Mittel.

**14.** Heilmittel enthaltend eine therapeutisch wirksame Menge eines 1H-Imidazols nach einem der Ansprüche 1 bis 11 oder eines seiner Additions-Salze von nicht toxischen, pharmazeutisch annehmbaren Säuren in Verbindung mit festen oder flüssigen pharmazeutischen Hilfstoffen.

**Patentanspruch für folgende Vertragsstaaten : ES, GR**

**1.** Verfahren zur Herstellung von substituierten 1H-Imidazolen, deren optischen Isomeren und deren racemischen Mischungen, der allgemeinen Formel:

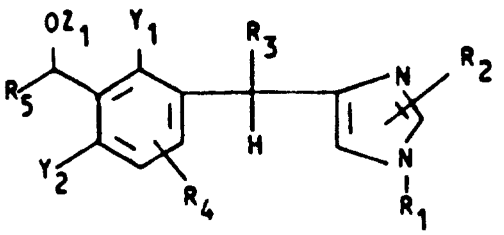

(I)

in der

| | |
|---|---|
| $R_1$, $R_2$, $R_3$ und $R_5$, | die gleich oder unterschiedlich sein können, jeweils ein Wasserstoffatom oder einen Alkylrest mit 1 bis 4 Kohlenstoffatomen darstellen, |
| $R_4$ | ein Wasserstoffatom, einen Alkylrest mit 1 bis 4 Kohlenstoffatomen oder einen Alkoxyrest mit 1 bis 4 Kohlenstoffatomen darstellt, |
| einer der Reste $Y_1$ und $Y_2$ | Wasserstoff und der andere einen $OZ_2$-Rest darstellt, und |
| $Z_1$ und $Z_2$ | getrennt genommen, alle beide Wasserstoff oder einen Alkylrest mit 1 bis 4 Kohlenstoffatomen und zusammen eine $-CH_2-$ oder $-C(CH_3)_2-$ Gruppe darstellen, |

sowie deren Additions-Salze von nicht toxischen, pharmazeutisch annehmbaren Säuren, dadurch gekennzeichnet, daß man

a) zur Herstellung der 1H-Imidazole der allgemeinen Formel I in der $Z_1$ und $Z_2$, getrennt genommen, einen Alkylrest mit 1 bis 4 Kohlenstoffatomen darstellen und, zusammen genommen, eine $-CH_2$ - oder $-C(CH_3)_2-$ Gruppe darstellen, eine Imidazolverbindung der Formel

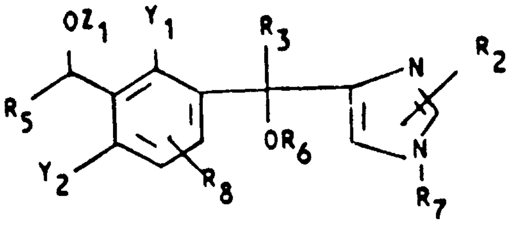

(II)

reduziert, in der $R_2$, $R_3$ und $R_5$ die oben genannte Bedeutung aufweisen, $R_6$ ein Wasserstoffatom oder einen Alkylrest mit 1 bis 4 Kohlenstoffatomen darstellt, $R_7$ einen $R_1$-Rest wie oben definiert oder einen leicht durch Reduktion abtrennbaren Rest darstellt, $R_8$ einen $R_4$-Rest wie oben definiert oder ein Chloratom darstellt, einer der Reste $Y_1$ und $Y_2$ Wasserstoff und der andere einen $OZ_2$-Rest darstellt, wobei $Z_1$ und $Z_2$ die oben genannte Bedeutung haben; oder

b) zur Herstellung der 1H-Imidazole der allgemeinen Formel I in der $Z_1$ und $Z_2$ Wasserstoff sind, in wässrigem, saurem Medium ein 4-[[2,2 Dimethyl-4H-1,3-benzodioxin-6(oder 8)-yl]methyl]-1H-imidazol der Formel

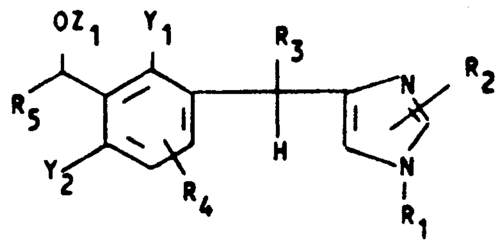

(III)

hydrolysiert, in der $R_1$, $R_2$, $R_3$, $R_4$ und $R_5$ die oben genannte Bedeutung aufweisen, einer der Reste $Y_1$ und $Y_2$ Wasserstoff und der andere einen $OZ_2$-Rest darstellt, wobei $Z_1$ und $Z_2$ zuzammen die -C-$(CH_3)_2$- Gruppe sind; oder

c) zur Herstellung der 1H-Imidazole der allgemeinen Formel I in der $Y_1 = OZ_2$ und $Y_2$, $Z_1$, $Z_2$ und $R_5$ Wasserstoff sind, ein 3-[(1H-Imidazol-4-yl)methyl]-2-hydroxybenzoësäurealkylester der Formel

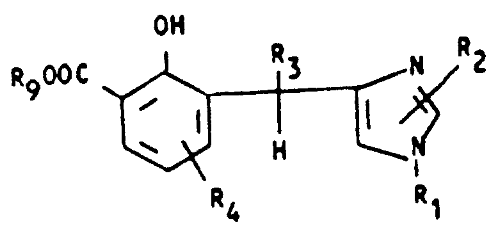

(IV)

reduziert, in der $R_1$, $R_2$, $R_3$ und $R_4$ die oben genannte Bedeutung aufweisen und $R_9$ einen Alkylrest mit 1 bis 4 Kohlenstoffatomen darstellt; und

d) gegebenfalls, die so erhaltenen 1H-Imidazole der Formel I in ihre Additions-Salze von nicht toxischen, pharmazeutisch annehmbaren Säuren umwandelt.